# EUROPEAN PATENT APPLICATION

(11) **EP 2 457 907 A1**
(43) Date of publication of application: **30.05.2012**
(21) Application number: 10306261.8
(22) Date of filing: 16.11.2010
(51) Int. Cl.: C07D 307/93, A61K 31/343, A61P 25/16, A61P 35/00

(54) **Flavagline derivatives as neuroprotective agents**

(71) Applicant: Université de Strasbourg, 67000 Strasbourg (FR); Université Pierre et Marie Curie - Paris 6, 75005 Paris (FR); Centre National de la Recherche Scientifique, 75016 Paris (FR)
(72) Inventor: Desaubry, Laurent, 67400 Illkirch (FR); Thuaud, Frédéric, 67400 Illkirch (FR); Ribeiro, Nigel, 67200 Strasbourg (FR); Bernard, Yohann, 57000 Metz (FR); Nebigil-Desaubry, Gunsel Canan, 67400 Illkirch (FR); Cresteil, Thierry, 92290 Chatenay Malabry (FR); Gaiddon, Christian, 67000 Strasbourg (FR); Hirsch, Etienne, 78000 Versailles (FR)
(74) Representative: Gallois, Valérie

(57) **Abstract**

The present invention discloses new flavagline derivatives (I) and the use of these compounds as neuroprotective and/or cardioprotective and/or antitumoral agents. In particular, the present invention discloses the use of these flavagline derivatives to prevent or limit the neurotoxicity of an antineoplastic agent. The use of these new compounds to prevent or treat a neurodegenerative disease of the nervous system, in particular Parkinson's disease, is also disclosed.

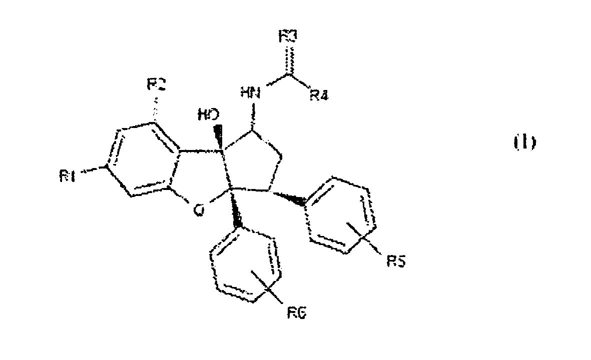

## Description

### Field of the Invention

The present invention relates to new flavaglines derivatives and pharmaceutical compositions comprising the same. The invention also relates to the prevention of antineoplastic agent neurotoxicity and/or cardiotoxicity by using these new flavagline derivatives as neuroprotective and/or cardioprotective agents. The invention further relates to the use of these compounds for the prevention or the treatment of neurodegenerative diseases of the nervous system.

### Background of the Invention

The use of anticancer agents in human therapy may cause a large number of toxic or side effects which may be life-threatening for the patients. These complications may lead to a reduction in the doses of the anticancer agents, and even to discontinuation of the therapy itself. Neurotoxicity is a common and often dose-limiting complication of chemotherapy treatment. Indeed, chemotherapeutic agents, in particular drugs belonging to platinum or taxane families, can cause toxic effects on peripheral nerves resulting from neuronal apoptosis. Severity may range from loss of sensory function and mild paresthesias to neuropathic pain, severe ataxia and weakness leading to pronounced disability. In many cases these neuropathies effectively limit the amount and the duration of chemotherapy given to patients. A regression of the neuropathy can be observed between cycles of the treatment or after treatment withdrawal. However, recovery is frequently incomplete and the neuropathy can permanently impair the patients' quality of life. Preventing or reducing this neurotoxicity is thus of extreme importance in the treatment of cancer. Various attempts have been made to prevent chemotherapy-induced neuropathy. However, despite intensive efforts, presently, no therapeutic options are available once a patient develops chemotherapy-induced neuropathy. The current strategies in neuroprotection clinical trials are based either on symptomatic treatment using antiepileptic or pain-killer drugs, such as pregabalin, lamotrigine and gabapentin, or on the administration of antioxidants, detoxicants, growth factors or growth-factor modulators (Kaley and Deangelis, 2009; Walker and Ni, 2007).

In addition to peripheral neuropathy induced by chemotherapy, numerous pathological conditions may result from death of neurons. Among them, Parkinson's disease is a common progressive neurodegenerative disease of the nervous system that afflicts patients later in life with tremor, slowness of movement, gait instability, and rigidity. These motor deficits have been mainly attributed to the progressive loss of dopaminergic neurons in the substantia nigra, and consequently the focus of current therapy has been on the replacement of dopamine. Even if this approach can substantially improve both the duration and quality of life of patients affected by Parkinson's disease, most patients develop late-stage motor and non-motor complications which are difficult to control with currently available treatments.

Flavaglines are a family of natural compounds extracted from Asian plants of the genus *Aglaia* (Kim et al., 2006; Proksch et al., 2001), comprising, for instance, rocaglaol, rocaglamide or silvestrol. It has been shown that some of these flavagline compounds may exhibit anticancer, immunosuppressive, cardioprotective (WO 2010/060891), neuroprotective (Fahrig et al., 2005; WO 03/045375), antifungal insecticide and antiinflammatory (EP 1 693 059) properties.

However, there is still a strong need of developing effective neuroprotective agents that are able to prevent or limit the neuronal cell death. In particular, there is a strong need of developing neuroprotective agents that do not reduce the antitumor activity of the anticancer chemotherapy and thus that could be used to prevent or to limit the neurotoxicity of these drugs.

### Summary of the Invention

The object of the present invention is to provide new neuroprotective agents. Preferably these neuroprotective agents also exhibit a cardioprotective activity and do not reduce the antitumor activity of the anticancer chemotherapy when administered to prevent or limit the neurotoxicity of such drugs. The inventors have even demonstrated that these compounds exhibit a significant cytotoxicity on numerous cancer cell lines.

Accordingly, in a first aspect, the present invention concerns a new flavagline derivative of the formula (I) 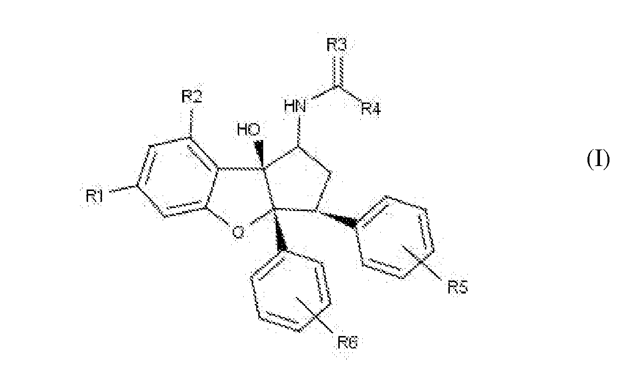 wherein
R¹ is alkoxy, optionally substituted, preferably selected from the group consisting of methoxy and a group -O-(CH₂)n-R⁷ wherein n is 1, 2, 3 or 4 and R⁷ is selected from the group consisting of hydroxyl, -NMe₂, -OCONMe₂, -OCONH₂ and morpholine;
R² is selected from the group consisting of hydrogen and (C₁-C₃)-alkoxy, preferably from hydrogen and methoxy;
R³ is selected from the group consisting of oxygen and sulphur;
R⁴ is selected from the group consisting of hydrogen, (C₁-C₃)-alkyl, optionally substituted, (C₁-C₃)-alkoxy, optionally substituted, (C₁-C₃)-hydroxyalkyl, a group - (CH₂)ₘ-N-R⁸R⁹, wherein m is 0, 1, 2 or 3, R⁸ and R⁹ are independently selected from the group consisting of hydrogen and (C₁-C₃)-alkyl;
R⁵ is selected from the group consisting of hydrogen and halogen, preferably from hydrogen and F;
R⁶ is halogen, preferably Br or Cl;
with the proviso that when R⁴ is hydrogen, then R⁵ is halogen,
or an enantiomer thereof; or an enantiomeric mixture thereof; or any pharmaceutically acceptable salt thereof.

In an embodiment, the flavagline derivative presents the formula (II) 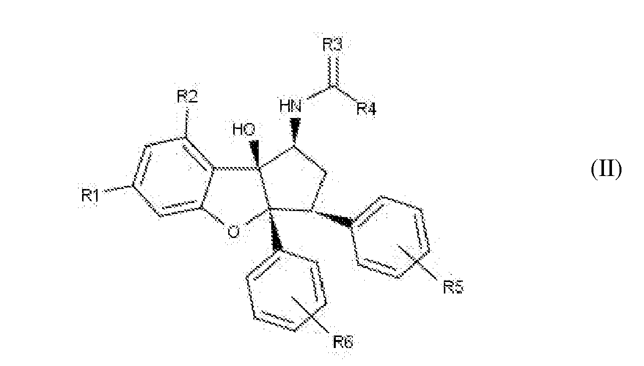 wherein the definitions of R¹, R², R³, R⁴, R⁵ and R⁶ are the same as above.

In a particular embodiment, R¹ and R² are (C₁-C₃)-alkoxy, preferably methoxy, and R³ is oxygen.

In another particular embodiment, R⁴ is selected from the group consisting of hydrogen, methyl, ethyl, methoxy, -CH₂OH, -NH₂**,** -NMe₂ and -CH₂NMe₂.

In another particular embodiment, R⁵ is halogen, preferably F, and is in meta position, and R⁶ is selected from the group consisting of Br and Cl and is in para position.

In another particular embodiment, R¹ and R² are methoxy; R³ is oxygen; R⁴ is selected from the group consisting of hydrogen, methyl, ethyl, methoxy, -CH₂OH, -NH₂, - NMe₂ and -CH₂NMe₂; R⁵ is F and is in meta position; and R⁶ is selected from the group consisting of Br and Cl and is in para position.

In another particular embodiment, R¹ and R² are methoxy; R³ is oxygen; R⁴ is selected from the group consisting of methyl, ethyl, methoxy, -CH₂OH, -NH₂, -NMe₂ and - CH₂NMe₂, preferably selected from the group consisting of methyl and ethyl; R⁵ is hydrogen; and R⁶ is selected from the group consisting of Br and Cl and is in para position.

In a second aspect, the present invention concerns a flavagline derivative of the invention as a medicament.

In a third aspect, the present invention concerns a pharmaceutical composition comprising a flavagline derivative of the invention and a pharmaceutically acceptable carrier and/or excipient.

In another aspect, the present invention concerns a pharmaceutical composition comprising a flavagline derivative of the invention and an antineoplastic agent.

In a further aspect, the present invention concerns a product containing a flavagline derivative of the invention and an antineoplastic agent, as a combined preparation for simultaneous, separate or sequential use, preferably in the treatment of cancer.

In a last aspect, the present invention concerns a flavagline derivative of the formula (I) 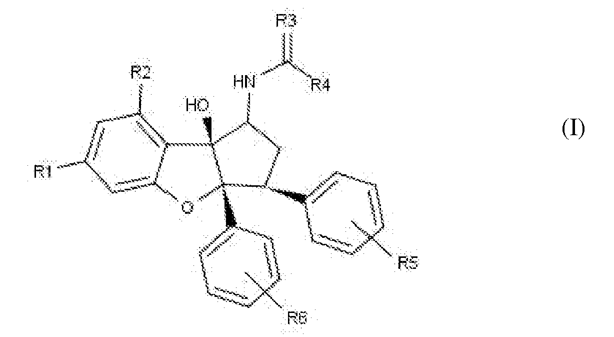 wherein
R¹ is alkoxy, optionally substituted, preferably selected from the group consisting of methoxy and a group -O-(CH₂)n-R⁷ wherein n is 1, 2, 3 or 4 and R⁷ is selected from the group consisting of hydroxyl, -NMe₂, -OCONMe₂, -OCONH₂ and morpholine;
R² is selected from the group consisting of hydrogen and (C₁-C₃)-alkoxy, preferably from hydrogen and methoxy;
R³ is selected from the group consisting of oxygen and sulphur;
R⁴ is selected from the group consisting of hydrogen, (C₁-C₃)-alkyl, optionally substituted, (C₁-C₃)-alkoxy, optionally substituted, (C₁-C₃)-hydroxyalkyl, a group -
(CH₂)ₘ-N-R⁸R⁹, wherein m is 0, 1, 2 or 3, R⁸ and R⁹ are independently selected from the group consisting of hydrogen and (C₁-C₃)-alkyl;
R⁵ is selected from the group consisting of hydrogen and halogen, preferably from hydrogen and F;
R⁶ is halogen, preferably Br or Cl;
or an enantiomer thereof; or an enantiomeric mixture thereof; or any pharmaceutically acceptable salt thereof, for use for the prevention or treatment of a neuropathy resulting from neuronal cell death, preferably a neuropathy selected from the group consisting of a neurodegenerative condition, a neuropathy induced by a neurotoxic agent and a neuropathy resulting from diabetes or HIV infection.

In a particular embodiment, the flavagline derivative presents the formula (II) 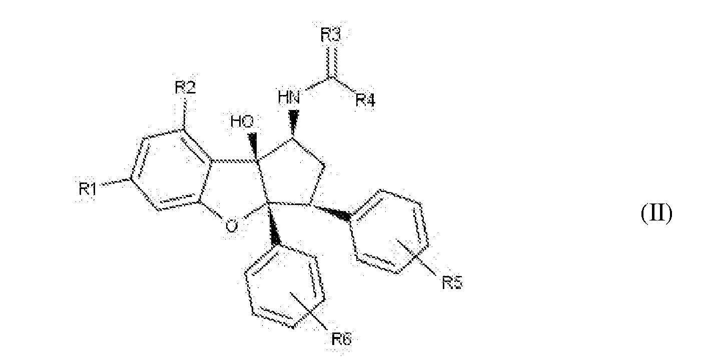 wherein the definitions of R¹ ,R², R³, R⁴, R⁵ and R⁶ are the same as above.

In a preferred embodiment, R¹ and R² are methoxy; R³ is oxygen; R⁴ is selected from the group consisting of hydrogen, methyl, ethyl, methoxy, -CH₂OH, -NH₂, -NMe₂ and - CH₂NMe₂; R⁵ is selected from the group consisting of hydrogen and halogen, preferably halogen; and R⁶ is halogen, preferably Br or Cl.

In another preferred embodiment, the neuropathy is a neuropathy induced by a neurotoxic agent and the neurotoxic agent is an antineoplastic agent.

### Legends of the Figures

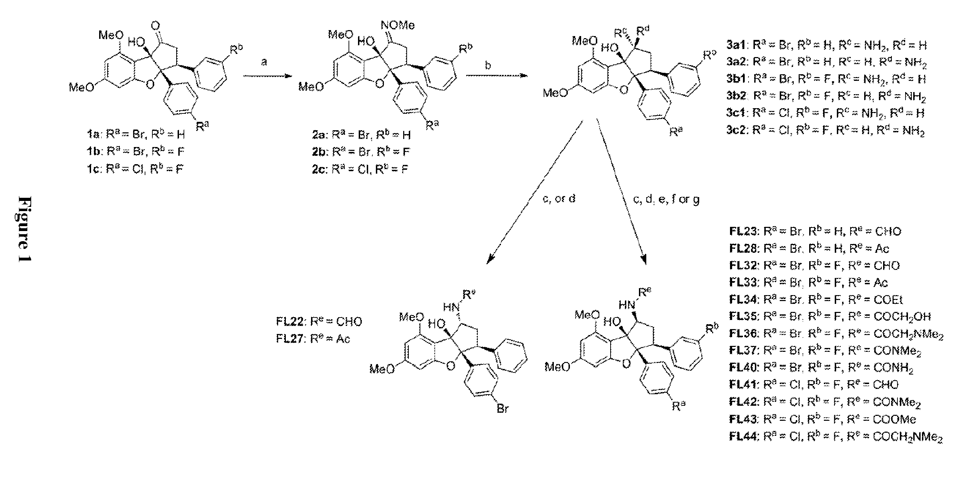

**Figure 1****:** Synthesis of flavagline derivatives. Reactants and conditions: (a) H₂NOMe.HCI, pyr, EtOH, 70 °C, 4 h; (b) BH₃·THF , THF, 66°C, 12 h; (c) HCOOEt, CH₃COOH, THF, reflux, 12 h; (d) Ac₂0 or (EtCO)₂O, DMAP, pyr, rt; (e) glycolic acid or dimethylglycine hydrochloride, EDCI, HOBt, DIPEA, DMF, rt; (f) dimethylcarbamoyl chloride or methyl chloroformate, DIPEA, DMAP, CH₂Cl₂, rt; (g) TMSNCO, CH₂Cl₂, iPrOH, rt.

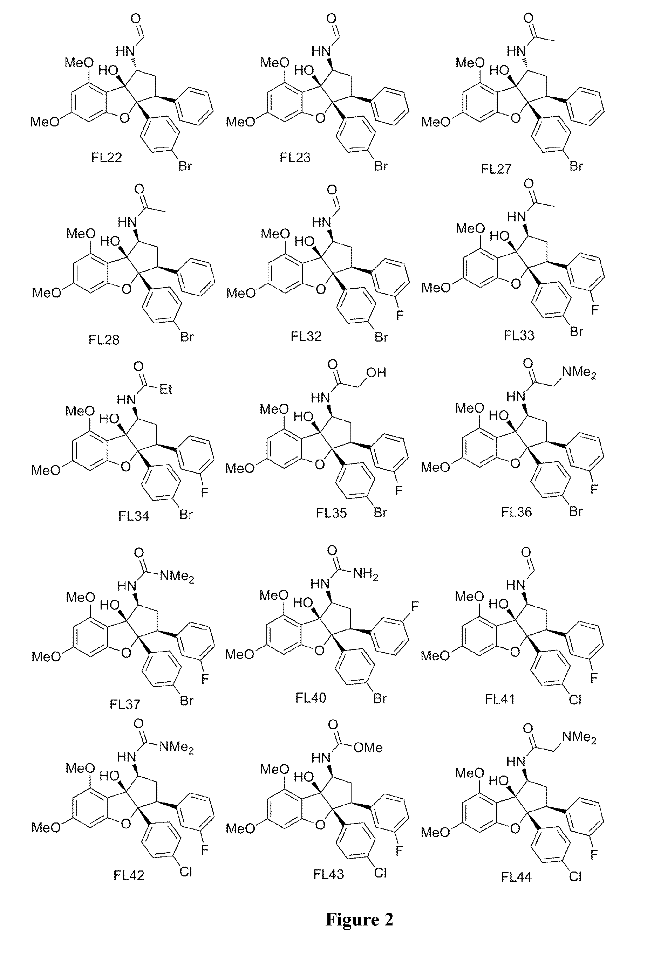

**Figure 2****:** Formulas of flavagline derivatives FL22, FL23, FL27, FL28, FL33 to FL37 and FL40 to FL44.

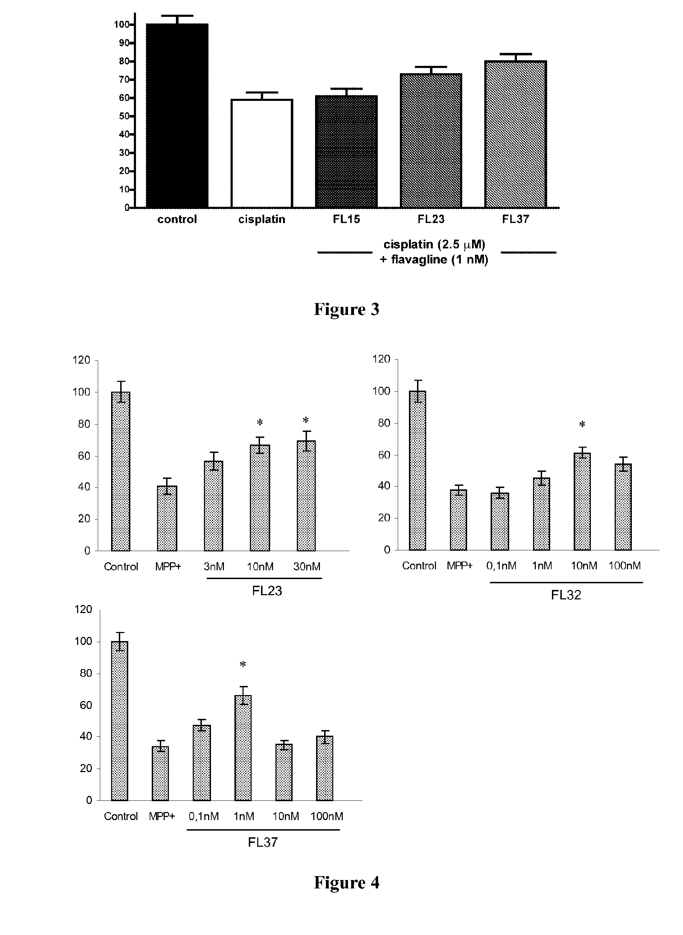

**Figure 3****:** Protection of neurons by rocaglaol derivative FL15 (previously described in the international patent application WO 03/045375), and flavagline derivatives FL23 and FL37 (1 nM) against apoptosis induced by cisplatin (2.5 µM). Bars indicate number of live cell relative to the vehicle.
**Figure 4****:** Effects of flavagline derivatives FL23, FL32 and FL37 on MPP⁺-treated primary mesencephalic neurons. Primary rat mesencephalic neurons were treated by 3µM MPP⁺ in the presence or absence of FL compounds. Dopaminergic neurons were detected by immunostaining tyrosine hydroxylase (TH) and counted using image analysis (*P<0.001).

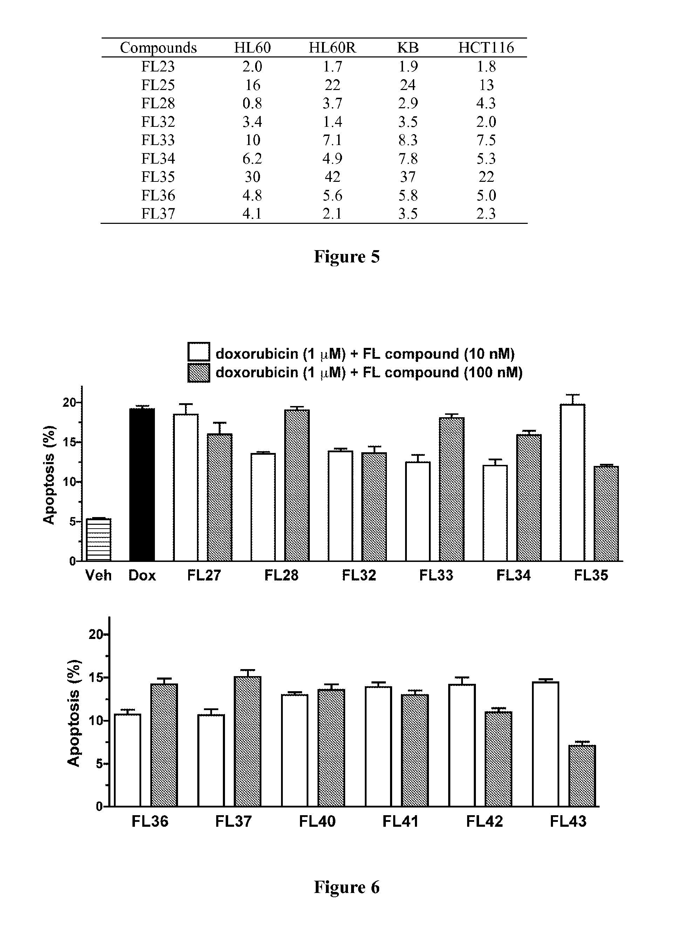

**Figure 5****:** Cytotoxicity of flavagline derivatives against human cancer cell lines (IC₅₀, nM, Data is the average of two independent IC₅₀ value determinations).
**Figure 6****:** *In vitro* cardioprotectant activity of flavagline derivatives on H9c2 cardiomyocytes against apoptosis induced by 1 µM doxorubicin. H9c2 cells were pretreated with flavagline derivatives (10 or 100 nM) or their vehicle for 10 h, then treated with doxorubicin (1 µM) for 14h additional hours and apoptosis was measured by FACS (annexin V and PI staining).

### Detailed description of the invention

The inventors disclose herein new flavagline derivatives that are able to prevent or limit the neuronal cell death (a) induced by antineoplastic agents such as cisplatin and (b) in a model of Parkinson's disease with improved efficiency. Furthermore, they have demonstrated that these compounds exhibit a significant cytotoxicity on numerous cancer cell lines. The inventors have further shown that, in addition to a neuroprotective effect, these flavagline derivatives also exhibit a cardioprotective effect.

### Definitions

As used herein, the term "alkoxy" corresponds to an alkyl group bonded to the molecule by an -O- (ether) bond. (C₁-C₃)-alkoxy groups include methoxy, ethoxy, propyloxy, and isopropyloxy.

The term "alkyl" as used herein, refers to a univalent radical containing only carbon and hydrogen atoms arranged in a chain. (C₁-C₃)-alkyl groups include methyl, ethyl, propyl, or isopropyl. The term "Me" as used herein, refers to a methyl group. The term "Et" as used herein, refers to an ethyl group.

The term "(C₁-C₃) hydroxyalkyl" comprises hydroxymethyl, hydroxyethyl, hydroxypropyl and hydroxyisopropyl.

The term "morpholin" as used herein, refers to a heterocycle of formula - O(CH₂CH₂)₂NH with both amine and ether functional groups.

The term "pharmaceutically acceptable salt" refers to salts which are non-toxic for a patient and suitable for maintaining the stability of a therapeutic agent and allowing the delivery of said agent to target cells or tissue. Pharmaceutically acceptable salts are well known in the art (Berge et al., 1977). These salts can be prepared in situ during the final isolation and purification of the flavagline derivatives, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, 3-phenylpropionate, phosphate, picrate, pivalate, propionate, stearate, succinate, sulfate, tartrate, thiocyanate, p-toluenesulfonate, undecanoate, valerate salts, and the like.

The term "enantiomeric mixture", as used herein, may refer to a racemic mixture, i.e. comprising equal amounts of left- and right-handed enantiomers, or to a mixture enriched in one of the enantiomers.

As used herein, the term "neuropathy resulting from neuronal cell death" may include any pathological condition resulting from neuronal cell death, in particular from neuronal cell apoptosis. The terms "neuronal cell", "nerve cell" and "neuron" are used herein interchangeably.

As used herein, the term "antineoplastic agent" refers to an agent with anti-cancer activity that inhibits or halts the growth of cancerous cells or immature pre-cancerous cells, kills cancerous cells or immature pre-cancerous cells, increases the susceptibility of cancerous or pre-cancerous cells to other antineoplastic agents, and/or inhibits metastasis of cancerous cells. These agents may include chemical agents as well as biological agents.

As used herein, the term "neuroprotective agent" refers to an agent that protects neuronal cells from damages, in particular from apoptosis.

As used herein, the term "cardioprotective agent" refers to an agent that protects cardiomyocytes from damages, in particular from apoptosis.

As used herein, the term "patient" or "subject" refers to any mammal, preferably a human being.

### New flavagline derivatives

In a first aspect, the present invention concerns new flavagline derivatives of the formula (I) 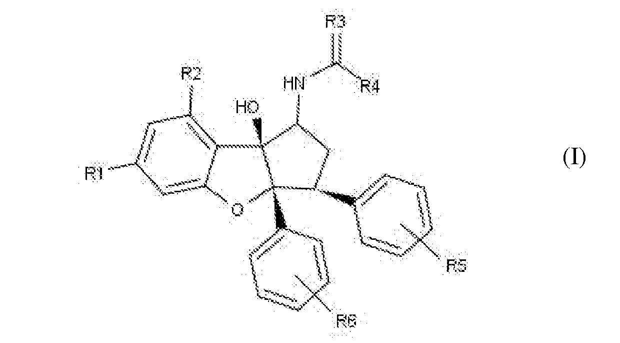 wherein
R¹ is alkoxy, optionally substituted, preferably selected from the group consisting of methoxy and a group -O-(CH₂)n-R⁷ wherein n is 1, 2, 3 or 4 and R⁷ is selected from the group consisting of hydroxyl, -NMe₂, -OCONMe₂, -OCONH₂ and morpholine;
R² is selected from the group consisting of hydrogen and (C₁-C₃)-alkoxy, preferably from hydrogen and methoxy;
R³ is selected from the group consisting of oxygen and sulphur;
R⁴ is selected from the group consisting of hydrogen, (C₁-C₃)-alkyl, optionally substituted, (C₁-C₃)-alkoxy, optionally substituted, (C₁-C₃)-hydroxyalkyl, a group - (CH₂)m-N-R⁸R⁹, wherein m is 0, 1, 2 or 3, R⁸ and R⁹ are independently selected from the group consisting of hydrogen and (C₁-C₃)-alkyl;
R⁵ is selected from the group consisting of hydrogen and halogen, preferably from hydrogen and F;
R⁶ is halogen, preferably Br or Cl;
with the proviso that when R⁴ is hydrogen, then R⁵ is halogen;
or an enantiomer thereof, or an enantiomeric mixture thereof; or any pharmaceutically acceptable salt thereof.

Alternatively, the present invention concerns new flavagline derivatives of the formula (I) wherein R¹, R², R³ and R⁶ are as disclosed above, and
R⁴ is hydrogen and R⁵ is halogen; or
R⁴ is selected from the group consisting of (C₁-C₃)-alkyl, optionally substituted, (C₁-C₃)-alkoxy, optionally substituted, (C₁-C₃)-hydroxyalkyl, a group -(CH₂)ₘ₋N-R⁸R⁹ wherein m is 0, 1, 2 or 3, R⁸ and R⁹ are independently selected from the group consisting of hydrogen and (C₁-C₃)-alkyl; and R⁵ is selected from the group consisting of hydrogen and halogen, preferably from hydrogen and F.

The flavagline derivatives of the present invention may be in an enantiomerically pure form or in an enantiomeric mixture.

In a particular embodiment, flavagline derivatives have the formula (II) 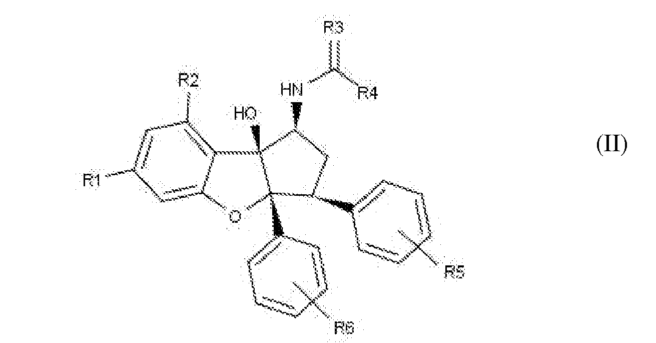 wherein the definitions of R¹ R², R³, R⁴, R⁵ and R⁶ are as disclosed above.

In another particular embodiment, flavagline derivatives have the formula (III) 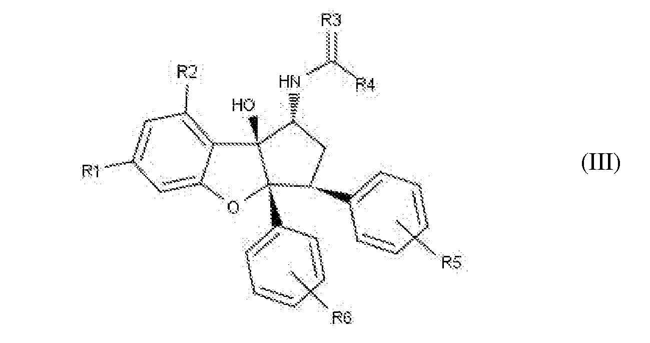 wherein the definitions of R¹, R², R³, R⁴, R⁵ and R⁶ are as disclosed above.

In an embodiment, the flavagline derivative of formula (I), (II) or (III) has one or several of the following features:
a) R¹ and R² are (C₁-C₃)-alkoxy;
b) R³ is oxygen;
c) R⁴ is selected from the group consisting of hydrogen, (C₁-C₃)-alkyl, (C₁-C₃)-alkoxy, (C₁-C₃)-hydroxyalkyl, a group -(CH₂)ₘ-N-R⁸R⁹, wherein m is 0, 1 or 2, R⁸ and R⁹ are independently selected from the group consisting of hydrogen and (C₁-C₃)-alkyl;
d) R⁵ is selected from the group consisting of hydrogen and halogen; and
e) R⁶ is halogen.

In a particular embodiment, the flavagline derivative of formula (I), (II) or (III) has one or several of the following features:
a) R¹ and R² are methoxy;
b) R³ is oxygen;
c) R⁴ is selected from the group consisting of hydrogen, methyl, ethyl, methoxy, - CH₂OH -NH₂, -NMe₂ and -CH₂NMe₂;
d) R⁵ is selected from the group consisting of hydrogen and fluorine; and
e) R⁶ is selected from the group consisting of Br and Cl.

In another particular embodiment, the flavagline derivative of formula (I), (II) or (III) has one or several of the following features:
a) R¹ and R² are methoxy;
b) R³ is oxygen;
c) R⁴ is selected from the group consisting of hydrogen, methyl, ethyl, methoxy, - CH₂OH, -NH₂, -NMe₂ and -CH₂NMe₂;
d) R⁵ is selected from the group consisting of hydrogen and fluorine and is in meta position; and
e) R⁶ is selected from the group consisting of Br and Cl and is in para position.

In another particular embodiment, the flavagline derivative of formula (I), (II) or (III) has one or several of the following features:
a) R¹ and R² are methoxy;
b) R³ is oxygen;
c) R⁴ is selected from the group consisting of hydrogen, methyl, ethyl, methoxy, - CH₂OH, -NH₂, -NMe₂ and -CH₂NMe₂;
d) R⁵ is fluorine and is in meta position; and
e) R⁶ is selected from the group consisting of Br and Cl and is in para position.

The flavagline derivative of formula (I), (II) or (III) may meet one of the above-listed features. It may also meet two of these features, for instance a) and b); a) and c); a) and d); a) and e); b) and c); b) and d); b) and e); c) and d); c) and e); or d) and e). The flavagline derivative may meet three of these features, for instance a), b) and c); a), b) and d); a), b) and e); a), c) and d); a), c) and e); a), d) and e); b), c) and d); b), c) and e); b), d) and e); or c), d) and e). The flavagline derivative may meet four of these features, for instance a), b), c) and d); a), b), c) and e); a), b), d) and e); b), c), d) and e); or a), c), d) and e). The flavagline derivative may also meet all of these features, i.e. a), b), c), d) and e).

In a particular embodiment, R¹ and R² are methoxy; R³ is oxygen; R⁴ is selected from the group consisting of hydrogen, (C₁-C₃)-alkyl, optionally substituted, (C₁-C₃)-alkoxy, optionally substituted, (C₁-C₃)-hydroxyalkyl, a group -(CH₂)ₘ₋N-R⁸R⁹, wherein m is 0, 1, 2 or 3, R⁸ and R⁹ are independently selected from the group consisting of hydrogen and (C₁-C₃)-alkyl; R⁵ is selected from the group consisting of hydrogen and halogen, preferably from hydrogen and F; R⁶ is halogen, preferably Br or Cl, with the proviso that when R⁴ is hydrogen, then R⁵ is halogen.

In a particular embodiment, R¹ and R² are methoxy; R³ is oxygen; R⁴ is hydrogen; R⁵ is halogen, preferably from hydrogen and F; R⁶ is halogen, preferably Br or Cl.

In another particular embodiment, R¹ and R² are methoxy; R³ is oxygen; R⁴ is selected from the group consisting of (C₁-C₃)-alkyl, optionally substituted, (C₁-C₃)-alkoxy, optionally substituted, (C₁-C₃)-hydroxyalkyl, a group -(CH₂)ₘ₋N-R⁸R⁹, wherein m is 0, 1, 2 or 3, R⁸ and R⁹ are independently selected from the group consisting of hydrogen and (C₁-C₃)-alkyl; R⁵ is selected from the group consisting of hydrogen and halogen, preferably from hydrogen and F; R⁶ is halogen, preferably Br or Cl.

In a particular embodiment, the flavagline derivative has the formula (II), wherein R¹ and R² are methoxy; R³ is oxygen; R⁴ is selected from the group consisting of hydrogen, methyl, ethyl, methoxy, -CH₂OH, -NMe₂, -NH₂ and -CH₂NMe₂; R⁵ is fluorine; and R⁶ is selected from the group consisting of bromine and chlorine.

In another particular embodiment, the flavagline derivative has the formula (II), wherein R¹ and R² are methoxy; R³ is oxygen; R⁴ is selected from the group consisting of hydrogen, methyl, methoxy, -CH₂OH, -NMe₂ and -CH₂NMe₂; R⁵ is selected from the group consisting of hydrogen and fluorine; and R⁶ is selected from the group consisting of bromine and chlorine, with the proviso that when R⁴ is hydrogen, then R⁵ is halogen.

In particular, the flavagline derivative has the formula (II), wherein R¹ and R² are methoxy; R³ is oxygen; and R⁶ is selected from the group consisting of bromine and chlorine; and R⁴ is hydrogen and R⁵ is halogen, or R⁴ is selected from the group consisting of methyl, methoxy, -CH₂OH, -NMe₂ and -CH₂NMe₂, and R⁵ is selected from the group consisting of hydrogen and fluorine.

In another particular embodiment, the flavagline derivative has the formula (II), wherein R¹ and R² are methoxy; R³ is oxygen; R⁴ is selected from the group consisting of methoxy, -CH₂OH, -NMe₂ and -CH₂NMe₂; R⁵ is fluorine; and R⁶ is selected from the group consisting of bromine and chlorine.

In another particular embodiment, the flavagline derivative has the formula (II), wherein R¹ and R² are methoxy; R³ is oxygen; R⁴ is selected from the group consisting of hydrogen, methyl and -NMe₂; R⁵ is selected from the group consisting of hydrogen and fluorine; and R⁶ is bromine, with the proviso that when R⁴ is hydrogen, then R⁵ is fluorine.

In particular, the flavagline derivative has the formula (II), wherein R¹ and R² are methoxy, R³ is oxygen and R⁶ is bromine; and R⁴ is hydrogen and R⁵ is fluorine, or R⁴ is selected from the group consisting of methyl and -NMe₂ and R⁵ is selected from the group consisting of hydrogen and fluorine.

In a more particular embodiment, the flavagline derivative has the formula (II), wherein R¹ and R² are methoxy; R³ is oxygen; R⁴ is selected from the group consisting of hydrogen and -NMe₂; R⁵ is fluorine; and R⁶ is bromine.

In another particular embodiment, the flavagline derivative has the formula (II), wherein R¹ and R² are methoxy; R³ is oxygen; R⁴ is hydrogen; R⁵ is fluorine; and R⁶ is bromine.

In a preferred embodiment, the flavagline derivative has the formula (II), wherein R¹ and R² are methoxy; R³ is oxygen; R⁴ is -NMe₂; R⁵ is fluorine; and R⁶ is bromine.

In another embodiment, the flavagline derivative has the formula (III), wherein R¹ and R² are methoxy; R³ is oxygen; R⁴ is (C₁-C₃)-alkyl, preferably methyl; R⁵ is hydrogen, and R⁶ is halogen, preferably bromine.

In a particular embodiment, the flavagline derivative is selected from the group consisting of FL27, FL28, FL32, FL33, FL34, FL35, FL36, FL37, FL40, FL41, FL 42, FL43 and FL44 as illustrated in Figure 2. In a preferred embodiment, the flavagline derivative is selected from the group consisting of FL28, FL32, FL35, FL36, FL37, FL42 and FL43.

The present invention also concerns a flavagline derivative according to the invention, as described above, as a medicament. The flavagline derivative can be a flavagline derivative according to any of the above embodiments and can optionally be selected from the group consisting of FL27, FL 28, FL32, FL33, FL34, FL35, FL36, FL37, FL40, FL41, FL 42, FL43 and FL44.

In a particular aspect, the present invention concerns a new flavagline derivative of the invention as neuroprotective agent, more particularly as neuroprotective agent against neurotoxicity of an antineoplastic agent. The present invention also concerns a flavagline derivative of the invention for use for preventing or limiting the neurotoxicity of a compound, in particular the neurotoxicity of an antineoplastic agent. It also concerns the use of a flavagline derivative of the invention for preparing a medicament for preventing or limiting the neurotoxicity of a compound, in particular the neurotoxicity of an antineoplastic agent. The present invention further concerns a method for preventing or limiting the neurotoxicity of a compound in a subject, in particular the neurotoxicity of an antineoplastic agent, comprising administering a therapeutically active amount of a flavagline derivative according to the invention. In particular, the derivative can be a flavagline derivative according to any of the above embodiments and can optionally be selected from the group consisting of FL27, FL 28, FL32, FL33, FL34, FL35, FL36, FL37, FL40, FL41, FL 42, FL43 and FL44. In a preferred embodiment, the derivative is selected from the group consisting of FL32 and FL37. More preferably the derivative is FL37.

In another particular aspect, the present invention concerns a flavagline derivative of the invention as cardioprotective agent, more particularly as cardioprotective agent against cardiotoxicity of an antineoplastic agent. The present invention also concerns a flavagline derivative of the invention for use for preventing or limiting the cardiotoxicity of a compound, in particular the cardiotoxicity of an antineoplastic agent. It also concerns the use of a flavagline derivative of the invention for preparing a medicament for preventing or limiting the cardiotoxicity of a compound, in particular the cardiotoxicity of an antineoplastic agent. The present invention further concerns a method for preventing or limiting the cardiotoxicity of a compound in a subject, in particular the cardiotoxicity of an antineoplastic agent, comprising administering a therapeutically active amount of a flavagline derivative according to the invention. In particular, in this aspect, the derivative can be a flavagline derivative according to any of the above embodiments and can optionally be selected from the group consisting of FL27, FL 28, FL32, FL33, FL34, FL35, FL36, FL37, FL40, FL41, FL 42, FL43 and FL44. In a preferred embodiment, the derivative is selected from the group consisting of FL35, FL36, FL37, FL42 and FL43.

In a further aspect, the present invention concerns a flavagline derivative of the invention as antitumoral agent. The present invention also concerns a flavagline derivative of the invention for use for treating cancer. It also concerns the use of a flavagline derivative of the invention for preparing a medicament for treating cancer. The present invention further concerns a method for treating a cancer in a subject, comprising administering a therapeutically active amount of a flavagline derivative according to the invention. In particular, in this aspect, the derivative can be a flavagline derivative according to any of the above embodiments and can optionally be selected from the group consisting of FL27, FL 28, FL32, FL33, FL34, FL35, FL36, FL37, FL40, FL41, FL42, FL43 and FL44. In a preferred embodiment, the derivative is selected from the group consisting of FL28, FL32 and FL37.

These flavagline derivatives can be synthesized by any process known by the man skilled in the art. Such processes are described, for example, in the patent applications DE 19934952 and WO 2005/092876 or in the articles of Diedrichs et al. (Diedrichs et al., 2005), Gerard et al. (Gerard et al., 2004) and Dobler et al. (Dobler et al., 2001). As illustrative example, the synthesis of the derivatives FL27, FL 28, FL32, FL33, FL34, FL35, FL36, FL37, FL40, FL41, FL42, FL43 and FL44 is detailed in the present application in the experimental section.

The present invention also concerns a pharmaceutical composition comprising a flavagline derivative of the invention, as described above, and a pharmaceutically acceptable carrier and/or excipient. This particular aspect also concerns the preferred embodiments disclosed above for the derivatives of the invention. In a particular embodiment, the pharmaceutical composition comprises a flavagline derivative according to any of the above embodiments. In a more particular embodiment, the pharmaceutical composition comprises a flavagline derivative selected from the group consisting of FL27, FL 28, FL32, FL33, FL34, FL35, FL36, FL37, FL40, FL41, FL42, FL43 and FL44. In another embodiment, the pharmaceutical composition comprises a flavagline derivative selected from the group consisting of FL28, FL32, FL35, FL36, FL37, FL42 and FL43. In a preferred embodiment, the pharmaceutical composition comprises a flavagline derivative selected from the group consisting of FL32 and FL37.

The pharmaceutical composition of the invention is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art. Possible pharmaceutical compositions include those suitable for oral, rectal, topical (including transdermal, buccal and sublingual), or parenteral (including subcutaneous, intramuscular, intravenous and intradermal) administration. For these formulations, conventional excipient can be used according to techniques well known by those skilled in the art. The compositions for parenteral administration are generally physiologically compatible sterile solutions or suspensions which can optionally be prepared immediately before use from solid or lyophilized form. Adjuvants such as a local anesthetic, preservative and buffering agents can be dissolved in the vehicle and a surfactant or wetting agent can be included in the composition to facilitate uniform distribution of the active ingredient. For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. For transdermal administration, the composition can be formulated into ointment, cream or gel form and appropriate penetrants or detergents could be used to facilitate permeation, such as dimethyl sulfoxide, dimethyl acetamide and dimethylformamide. For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. In a preferred embodiment, the pharmaceutical composition of the invention is suitable for parenteral administration.

Pharmaceutical composition according to the invention may be formulated to release the active drug substantially immediately upon administration or at any predetermined time or time period after administration.

In a particular embodiment, the pharmaceutical composition according to the invention comprises 0.1 mg to 1 g of the flavagline derivative of the invention. Preferably, pharmaceutical composition according to the invention comprises 0.7 mg to 700 mg of the flavagline derivative of the invention.

Pharmaceutical composition according to the invention can comprise one or more flavagline derivatives associated with pharmaceutically acceptable excipients and/or carriers. In a particular embodiment, the pharmaceutical composition comprise one or more flavagline derivatives selected from the group consisting of FL27, FL 28, FL32, FL33, FL34, FL35, FL36, FL37, FL40, FL41, FL 42, FL43 and FL44, associated with pharmaceutically acceptable excipients and/or carriers. These excipients and/or carriers are chosen according to the form of administration as described above.

Other active compounds can also be associated with flavagline derivatives of the present invention. For example, the derivatives can be associated with other neuroprotective agents; other cardioprotective agents; and/or drugs with neurotoxic and/or cardiotoxic side-effects such as antineoplastic agents or antiviral agents such as D4T (2'-3'-didehydro-2'-3'-dideoxythymidine or Stavudine), ddI (2'3'-dideoxyinosine or Didanosine) or ddC (2'-3'-dideoxycytidine or Zalcitabine).

In an embodiment, the pharmaceutical composition comprises one or more flavagline derivatives of the present invention associated with an antineoplastic agent, preferably a neurotoxic antineoplastic agent. Preferably, the antineoplastic agent is selected from the group consisting of antineoplastic agents belonging to platinum, taxane, anthracycline and vinca alkaloid families. More preferably the antineoplastic agent is selected from the group consisting of antineoplastic agents belonging to platinum and taxane families. In a particular embodiment, the antineoplastic agent is selected from the group consisting of cisplatin, carboplatin, oxaplatin, picoplatin, tetraplatin, satraplatin, paclitaxel, docetaxel, doxorubicin, daunorubicin, idarubicin, detorubicin, carminomycin, epirubicin, morpholinodoxorubicin, morpholinodaunorubicin, methoxymorpholinyldoxorubicin, vinblastine, vincristine, vindesine and vinorelbine, and derivatives and combinations thereof. In a more particular embodiment, the antineoplastic agent is selected from the group consisting of cisplatin, paclitaxel, docetaxel and doxorubicin. In a preferred embodiment, the antineoplastic agent is cisplatin. In a particularly preferred embodiment, the pharmaceutical composition comprises the derivative FL37 associated with cisplatin.

The present invention also concerns a product containing a flavagline derivative of the present invention and an antineoplastic agent, as a combined preparation for simultaneous, separate or sequential use in the treatment of cancer. The flavagline derivative can be a flavagline derivative according to any of the above embodiments. In a particular embodiment, the product contains one or more flavagline derivatives selected from the group consisting of FL27, FL 28, FL32, FL33, FL34, FL35, FL36, FL37, FL40, FL41, FL42, FL43 and FL44. In a particular embodiment, the product contains an antineoplastic agent selected from the group consisting of antineoplastic agents belonging to platinum, taxane, anthracycline and vinca alkaloid families, preferably from the group consisting of cisplatin, carboplatin, oxaplatin, picoplatin, tetraplatin, satraplatin, paclitaxel, docetaxel, doxorubicin, daunorubicin, idarubicin, detorubicin, carminomycin, epirubicin, morpholinodoxorubicin, morpholinodaunorubicin, methoxymorpholinyldoxorubicin, vinblastine, vincristine, vindesine and vinorelbine, and derivatives and combinations thereof. In a more particular embodiment, the product contains one or more flavagline derivatives selected from the group consisting of FL27, FL 28, FL32, FL33, FL34, FL35, FL36, FL37, FL40, FL41, FL42, FL43 and FL44, and an antineoplastic agent selected from the group consisting of cisplatin, carboplatin, oxaplatin, picoplatin, tetraplatin, satraplatin, paclitaxel, docetaxel, doxorubicin, daunorubicin, idarubicin, detorubicin, carminomycin, epirubicin, morpholinodoxorubicin, morpholinodaunorubicin, methoxymorpholinyldoxorubicin, vinblastine, vincristine, vindesine and vinorelbine, and derivatives and combinations thereof. In a particularly preferred embodiment, the product contains the derivative FL37 and the antineoplastic agent cisplatin.

### Prevention or treatment of neuropathy

In another aspect, the present invention concerns flavagline derivatives of the formula (I) 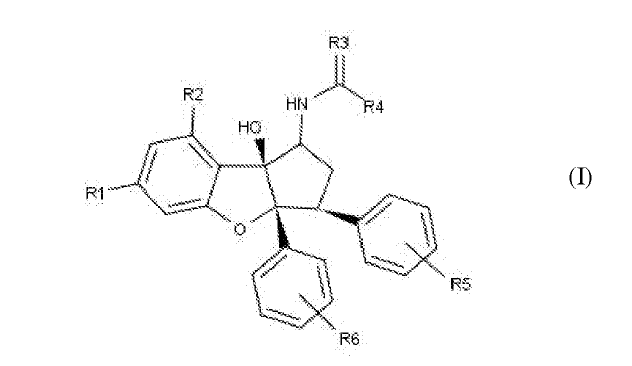 wherein
R¹ is alkoxy, optionally substituted, preferably selected from the group consisting of methoxy and a group -0-(CH₂)n-R⁷ wherein n is 1, 2, 3 or 4 and R⁷ is selected from the group consisting of hydroxyl, -NMe₂, -OCONMe_{2,} -OCONH₂ and morpholine;
R² is selected from the group consisting of hydrogen and (C₁-C₃)-alkoxy, preferably from hydrogen and methoxy;
R³ is selected from the group consisting of oxygen and sulphur;
R⁴ is selected from the group consisting of hydrogen, (C₁-C₃)-alkyl, optionally substituted, (C₁-C₃)-alkoxy, optionally substituted, (C₁-C₃)-hydroxyalkyl, a group - (CH₂)ₘ-N-R⁸R⁹, wherein m is 0, 1, 2 or 3, R⁸ and R⁹ are independently selected from the group consisting of hydrogen and (C₁-C₃)-alkyl;
R⁵ is selected from the group consisting of hydrogen and halogen, preferably from hydrogen and F;
R⁶ is halogen, preferably Br or Cl;
or an enantiomer thereof; or an enantiomeric mixture threrof; or any pharmaceutically acceptable salt thereof, for use for the prevention or treatment of a neuropathy resulting from neuronal cell death, in particular neuronal cell apoptosis.

The flavagline derivatives used in the present invention may be used in an enantiomerically pure form or in an enantiomeric mixture.

In a particular embodiment, flavagline derivatives have the formula (II) 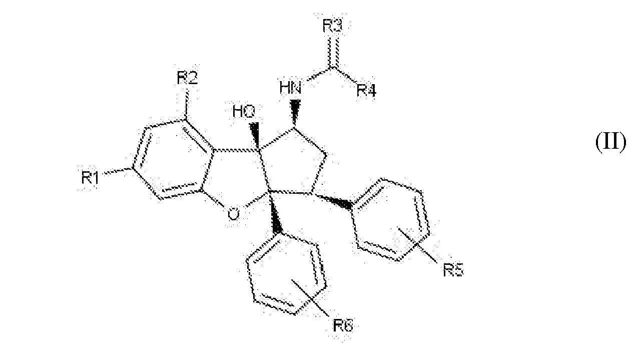 wherein the definitions of R¹, R², R³, R⁴, R⁵ and R⁶ are as disclosed above.

In another particular embodiment, flavagline derivatives have the formula (III) 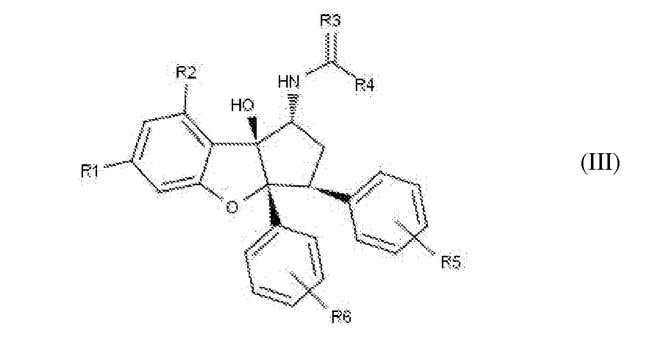 wherein the definitions of R¹, R² , R³, R⁴, R⁵ and R⁶ are as disclosed above.

In an embodiment, the flavagline derivative of formula (I), (II) or (III) has one or several of the following features:
a) R¹ and R² are (C₁-C₃)-alkoxy;
b) R³ is oxygen;
c) R⁴ is selected from the group consisting of hydrogen, (C₁-C₃)-alkyl, (C₁-C₃)-alkoxy, (C₁-C₃)-hydroxyalkyl, a group -(CH₂)ₘ-N-R⁸R⁹, wherein m is 0, 1 or 2, R⁸ and R⁹ are independently selected from the group consisting of hydrogen and (C₁-C₃)-alkyl;
d) R⁵ is selected from the group consisting of hydrogen and halogen; and
e) R⁶ is halogen.

In a particular embodiment, the flavagline derivative of formula (I), (II) or (III) has one or several of the following features:
a) R¹ and R² are methoxy;
b) R³ is oxygen;
c) R⁴ is selected from the group consisting of hydrogen, methyl, ethyl, methoxy, - CH₂OH, -NH₂, -NMe₂ and -CH₂NMe₂;
d) R⁵ is selected from the group consisting of hydrogen and fluorine; and
e) R⁶ is selected from the group consisting of Br and Cl.

The flavagline derivative of formula (I), (II) or (III) may meet one of these features. It may also meet two of these features, for instance a) and b); a) and c); a) and d); a) and e); b) and c); b) and d); b) and e); c) and d); c) and e); or d) and e). The flavagline derivative may meet three of these features, for instance a), b) and c); a), b) and d); a), b) and e); a), c) and d); a), c) and e); a), d) and e); b), c) and d); b), c) and e); b), d) and e); or c), d) and e). The flavagline derivative may meet four of these features, for instance a), b), c) and d); a), b), c) and e); a), b), d) and e); b), c), d) and e); or a), c), d) and e). The flavagline derivative may also meet all of these features, i.e. a), b), c), d) and e).

In a particular embodiment, the flavagline derivative has the formula (II), wherein R¹ and R² are methoxy; R³ is oxygen; R⁴ is selected from the group consisting of hydrogen, methyl, ethyl, methoxy, -CH₂OH -NMe₂, -NH₂ and -CH₂NMe₂; R⁵ is fluorine; and R⁶ is selected from the group consisting of bromine and chlorine.

In another particular embodiment, the flavagline derivative has the formula (II), wherein R¹ and R² are methoxy; R³ is oxygen; R⁴ is selected from the group consisting of hydrogen, methyl, methoxy, -CH₂OH, -NMe₂ and -CH₂NMe₂; R⁵ is selected from the group consisting of hydrogen and fluorine; and R⁶ is selected from the group consisting of bromine and chlorine.

In another particular embodiment, the flavagline derivative has the formula (II), wherein R¹ and R² are methoxy; R³ is oxygen; R⁴ is selected from the group consisting of hydrogen and -NMe₂; R⁵ is selected from the group consisting of hydrogen and fluorine; and R⁶ is bromine.

In a more particular embodiment, the flavagline derivative has the formula (II), wherein R¹ and R² are methoxy; R³ is oxygen; R⁴ is selected from the group consisting of hydrogen and -NMe₂; R⁵ is fluorine; and R⁶ is bromine.

In another particular embodiment, the flavagline derivative has the formula (II), wherein R¹ and R² are methoxy; R³ is oxygen; R⁴ and R⁵ are hydrogen; and R⁶ is bromine.

In another particular embodiment, the flavagline derivative has the formula (II), wherein R¹ and R² are methoxy; R³ is oxygen; R⁴ is hydrogen; R⁵ is fluorine; and R⁶ is bromine.

In a preferred embodiment, the flavagline derivative has the formula (II), wherein R¹ and R² are methoxy; R³ is oxygen; R⁴ is -NMe₂; R⁵ is fluorine; and R⁶ is bromine.

In another embodiment, the flavagline derivative has the formula (III), wherein R¹ and R² are methoxy; R³ is oxygen; R⁴ is selected from the group consisting of hydrogen and (C₁-C₃)-alkyl, preferably selected from the group consisting of hydrogen and methyl; R⁵ is hydrogen, and R⁶ is halogen, preferably bromine.

In a preferred embodiment, the flavagline derivative used in the invention is selected from the group consisting of FL22, FL23, FL27, FL28, FL32, FL 33, FL34, FL35, FL36, FL37, FL40, FL41, FL42, FL43 and FL44 (as illustrated in Figure 2).

The neuropathy resulting from neuronal cell death may be a neuropathy of the central nervous system (CNS) or a neuropathy of the peripheral nervous system (PNS). In an embodiment, the neuropathy resulting from neuronal cell death is selected from the group consisting of neurodegenerative conditions, neuropathies induced by a neurotoxic agent and neuropathies resulting from diabetes or HIV infection.

In an embodiment, the neuropathy is a neurodegenerative condition. This neurodegenerative condition may result from the death of selective neuronal populations, e.g. motor neurons in amyotrophic lateral sclerosis or dopaminergic neurons in Parkinson's disease. In a particular embodiment, the neurodegenerative condition is selected from the group consisting of Parkinson's disease and other parkinsonian disorders, Alzheimer's disease and other dementing neurodegenerative disorders, amyotrophic lateral sclerosis, multiple sclerosis, Creutzfeldt-Jakob disease, Huntington's disease and neuronal degeneration associated to multiple sclerosis. In a more particular embodiment, the neurodegenerative condition is selected from the group consisting of Parkinson's disease, Alzheimer's disease, amyotrophic lateral sclerosis, multiple sclerosis, Creutzfeldt-Jakob disease and Huntington's disease. In a preferred embodiment, the neurodegenerative condition is Parkinson's disease.

In another embodiment, the neuropathy is a neuropathy induced by a neurotoxic agent. The neurotoxic agent may be any compound known to induce neuronal cell death, and in particular neuronal cell apoptosis. Examples of such neurotoxic agents include, but are not limited to, drugs with neurotoxic side-effects such as some antineoplastic agents, antibiotics, anti-viral agents such as D4T, ddI or ddC; pesticidal carbamates; N-methyl-4-phenyl-1,2,3,6-tetrahydropyridine (MPTP); N-methyl-D-aspartic acid (NMDA); annonacine; rotenone; or paraquat. In a preferred embodiment, the neurotoxic agent is an antineoplastic agent with neurotoxic side effects. Examples of antineoplastic agents exhibiting neurotoxic side effects include, but are not limited to, platinum analogs such as cisplatin, carboplatin, oxaplatin, picoplatin, tetraplatin, satraplatin; taxanes such as paclitaxel (Taxol), docetaxel (Taxotere), larotaxel, cabazitaxel, BMS-184476, BMS-188797, BMS-275183, ortataxel, RPR 109881A, RPR 116258, NBT-287, Tesetaxel, IDN 5390, Taxoprexin and MAC-321; anthracyclines such as doxorubicin, daunorubicin, idarubicin, detorubicin, carminomycin, epirubicin, morpholinodoxorubicin, morpholinodaunorubicin, methoxymorpholinyldoxorubicin; vinca alkaloids such as vinblastine, vincristine, vindesine and vinorelbine; and derivatives and combinations thereof. In a particular embodiment, the neurotoxic agent is an antineoplastic agent belonging to platinum or taxane family. In a more particular embodiment, the neurotoxic agent is selected from the group consisting of cisplatin, carboplatin, oxaplatin, picoplatin, tetraplatin, satraplatin, paclitaxel and docetaxel, derivatives and combinations thereof. In a preferred embodiment, the neurotoxic agent is cisplatin.

In a further embodiment, the neuropathy results from diabetes or HIV infection. Examples of neuropathies resulting from diabetes or HIV infection include, but are not limited to, diabetic neuropathies and neuropathies associated with AIDS such as distal symmetrical polyneuropathy (DSPN), and acute or chronic inflammatory demyelinating polyneuropathy (AIDP/CIDP). Diabetic neuropathies are neuropathic disorders that are associated with diabetes mellitus. Preferably, the neuropathy resulting from diabetes or HIV infection is distal symmetrical polyneuropathy (DSPN).

In a particular embodiment, the neuropathy resulting from neuronal cell death is selected from the group consisting of neurodegenerative conditions and neuropathies induced by a neurotoxic agent. In another particular embodiment, the neuropathy resulting from neuronal cell death is selected from the group consisting of neurodegenerative conditions and neuropathies induced by an antineoplastic agent, preferably from the group consisting of Parkinson's disease and neuropathies induced by an antineoplastic agent.

As used herein, the term "prevention or treatment of a neuropathy" or "to prevent or to treat a neuropathy" refers to any act intended to ameliorate the health status of patients such as therapy, prevention, prophylaxis and retardation of the neuropathy. In certain embodiments, such term refers to the amelioration or eradication of the neuropathy or symptoms associated with said neuropathy. In other embodiments, this term refers to minimizing the spread or worsening of the neuropathy resulting from the administration of one or more therapeutic agents to a subject with said neuropathy.

The effect of the flavagline derivatives on the neuronal cell death, and in particular on the apoptosis of neuronal cells, can be assessed by any method known by the skilled person. For example, in the experimental section, the effects of compounds according to invention are assessed by measuring and comparing the death of neurons in absence and in presence of a neurotoxic agent, e.g. cisplatin or MPP⁺ (the active metabolite of the neurotoxin MPTP), and with a combination of the neurotoxic agent and a flavagline derivative of the invention. Preferably, the neuronal cells death in presence of a flavagline derivative of the invention is reduced by at least 5%, at least 10%, or at least 25%, more preferably by at least 30%, at least 40% or at least 50%.

In another aspect, the present invention concerns a method for preventing or treating a neuropathy resulting from neuronal cell death in a subject comprising administering a therapeutically effective amount of a flavagline derivative of the formula (I), (II) or (III) as described above, or an enantiomer thereof, or an enantiomeric mixture threrof; or any pharmaceutically acceptable salt thereof, to said subject. By a "therapeutically effective amount" is intended an amount of flavagline derivative of the invention administered to a patient that is sufficient to provide a therapeutic effect, i.e. that is sufficient to protect neuronal cells from damages, in particular from apoptosis. This therapeutically effective amount comprises a blood plasma concentration in a range of from 1 nM to 1 µM, preferably of from 15 nM to 100, more preferably of from 20 nM to 70 nM. Then, it is to be understood that the total amount of the flavagline derivative of the invention may vary depending on the volume of blood plasma of the patient. Suitable means and measures to determine the therapeutically effective amount are available to the person skilled in the art. In a particular embodiment, the method of the invention for preventing or treating a neuropathy resulting from neuronal cell death comprises administering 0.01 to 10 mg / kg of body weight / day of a flavagline derivative as described above or any pharmaceutically acceptable salt thereof to said subject. In a preferred embodiment, the flavagline derivative is selected from the group consisting of FL22, FL23, FL27, FL28, FL32, FL 33, FL34, FL35, FL36, FL37, FL40, FL41, FL42, FL43 and FL44, preferably from the group consisting of FL23, FL32 and FL37. All embodiments as disclosed above for flavagline derivatives for use for prevention or treatment of a neuropathy resulting from neuronal cell death are also encompassed in this aspect.

The present invention further concerns the use of a flavagline derivative as described above, or an enantiomer thereof, or an enantiomeric mixture threrof; or any pharmaceutically acceptable salt thereof, for preparing a medicament for preventing or treating a neuropathy resulting from neuronal cell death. In a preferred embodiment, the flavagline derivative is selected from the group consisting of FL22, FL23, FL27, FL28, FL32, FL 33, FL34, FL35, FL36, FL37, FL40, FL41, FL42, FL43 and FL44, preferably from the group consisting of FL23, FL32 and FL37. All embodiments as disclosed above for flavagline derivatives for use for prevention or treatment of a neuropathy resulting from neuronal cell death are also encompassed in this aspect.

In a particular aspect, the present invention concerns flavagline derivatives of the formula (I) 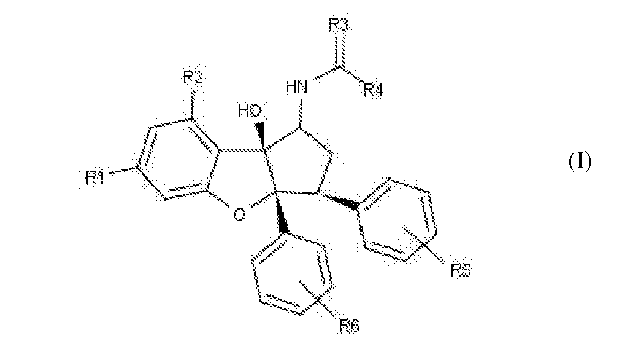 wherein
R¹ is alkoxy, optionally substituted, preferably selected from the group consisting of methoxy and a group -0-(CH₂)n-R⁷ wherein n is 1, 2, 3 or 4 and R⁷ is selected from the group consisting of hydroxyl, -NMe₂, -OCONMe₂, -OCONH₂ and morpholine;
R² is selected from the group consisting of hydrogen and (C₁-C₃)-alkoxy, preferably from hydrogen and methoxy;
R³ is selected from the group consisting of oxygen and sulphur;
R⁴ is selected from the group consisting of hydrogen, (C₁-C₃)-alkyl, optionally substituted, (C₁-C₃)-alkoxy, optionally substituted, (C₁-C₃)-hydroxyalkyl, a group - (CH₂)ₘ-N-R⁸R⁹, wherein m is 0, 1, 2 or 3, R⁸ and R⁹ are independently selected from the group consisting of hydrogen and (C₁-C₃)-alkyl;
R⁵ is selected from the group consisting of hydrogen and halogen, preferably from hydrogen and F;
R⁶ is halogen, preferably Br or Cl;
or an enantiomer thereof, or an enantiomeric mixture thereof; or any pharmaceutically acceptable salt thereof, for use to prevent or to limit the neurotoxicity of an antineoplastic agent. In a preferred embodiment, the flavagline derivative can be any compound of formula (I), (II) or (III) disclosed herein and is optionally selected from the group consisting of FL22, FL23, FL27, FL28, FL32, FL 33, FL34, FL35, FL36, FL37, FL40, FL41, FL42, FL43 and FL44, preferably from the group consisting of FL23, FL32 and FL37. All embodiments as disclosed above for flavagline derivatives for use for prevention or treatment of a neuropathy resulting from neuronal cell death are also encompassed in this aspect. As used herein, the term "to prevent or to limit the neurotoxicity" means inhibiting or reducing neurotoxic side effects of the antineoplastic agent administered to the subject, in particular, inhibiting or reducing the apoptosis of neuronal cells. In a particular embodiment, this term means inhibiting or reducing peripheral neuropathy induced by the antineoplastic agent administered to the subject.

The present invention further concerns the use of a flavagline derivative of the formula (I), (II) or (III) as described above, or an enantiomer thereof, or an enantiomeric mixture thereof; or any pharmaceutically acceptable salt thereof, for preparing a medicament for preventing or limiting the neurotoxicity of an antineoplastic agent. In a preferred embodiment, the flavagline derivative is selected from the group consisting of FL22, FL23, FL27, FL28, FL32, FL 33, FL34, FL35, FL36, FL37, FL40, FL41, FL42, FL43 and FL44, preferably from the group consisting of FL23, FL32 and FL37. All embodiments as disclosed above for flavagline derivatives for use for prevention or treatment of a neuropathy resulting from neuronal cell death are also encompassed in this aspect.

The present invention also concerns the use of a flavagline derivative of the formula (I), (II) or (III) as described above, or an enantiomer thereof, or an enantiomeric mixture thereof; or any pharmaceutically acceptable salt thereof, as neuroprotective agent. In a preferred embodiment, the flavagline derivative is selected from the group consisting of FL22, FL23, FL27, FL28, FL32, FL 33, FL34, FL35, FL36, FL37, FL40, FL41, FL42, FL43 and FL44, preferably from the group consisting of FL23, FL32 and FL37. All embodiments as disclosed above for flavagline derivatives for use for prevention or treatment of a neuropathy resulting from neuronal cell death are also encompassed in this aspect.

In another aspect, the present invention concerns a method for inhibiting neuronal cell death, in particular neuronal cell apoptosis, in a subject, comprising administering to a subject in need of such treatment a flavagline derivative of the formula (I), (II) or (III) as described above, in an amount effective to inhibit neuronal cell death in the subject. In a preferred embodiment, the flavagline derivative is selected from the group consisting of FL22, FL23, FL27, FL28, FL32, FL 33, FL34, FL35, FL36, FL37, FL40, FL41, FL42, FL43 and FL44, preferably from the group consisting of FL23, FL32 and FL37. All embodiments as disclosed above for flavagline derivatives for use for prevention or treatment of a neuropathy resulting from neuronal cell death are also encompassed in this aspect.

The following examples are given for purposes of illustration and not by way of limitation.

### Examples

### Synthesis and NMR characterization of flavagline derivatives

Ketones **1a-c** were synthesized according to an established strategy. They were transformed into oximes **2a-c** and reduced with borane affording diastereomeric mixture of amines **3**, which were acylated to afford target compounds (Figure 1).

The formulas of synthesized flavagline derivatives FL22, FL23, FL27, FL28, FL33 to FL37 and FL40 to FL44 are shown in Figure 2.

**3a-(4-Bromophenyl)-6,8-dimethoxy-8b-hydroxy-3-phenyl-2,3,3a,8b-tetrahydro-1*H*-cyclopenta[*b*]benzofuran-1-one 0-methyl oxime (2a)**. Ketone **1a** (500 mg, 1.04 mmol) and O-methylhydroxylamine hydrochloride (430 mg, 5.2 mmol) were diluted in absolute ethanol (20 mL) and distilled pyridine (20 mL). The mixture was concentrated in *vacuo,* extracted with EtOAc (10 mL), washed with HCI (2x10 mL, 1M), a saturated solution of Na₂C0₃ (10 mL), brine (10 mL) and dried over MgS0₄. Concentration to dryness quantitatively yielded the desired oxime 2a (530 mg) as a white solid, which was used in the next step without purification. ¹H NMR (CDC1₃): 3.07 (2H, m), 3.70 (1H, m), 3.82 (3H, s), 3.84 (3H, s), 4.06 (3H, s), 6.10 (1H, d, J = 2.0 Hz), 6.27 (1H, d, J = 2.0 Hz), 7.00 (4H, m), 7.08 (3H, m), 7.27 (2H, d, *J* = 8.5 Hz). ¹³C NMR (CDCl₃): 29.9, 49.9, 55.7, 55.9, 62.7, 87.5, 88.8, 93.1, 101.7, 108.2, 121.7, 127.0, 128.0 (2C), 128.1 (2C), 128.7 (2C), 130.9 (2C), 133.8 (2C), 137.5, 158.9, 160.2, 160.4, 164.4.

**3a-(4-Bromophenyl)-6,8-dimethoxy-(3-fluorophenyl)-8b-hydroxy-3-2,3,3a,8b-tetrahydro-1*H*-cyclopenta[*b*]benzofuran-1-one 0-methyl oxime (2b).** Ketone **1b** (500 mg, 1.00 mmol) and O-methylhydroxylamine hydrochloride (418 mg, 5.00 mmol) were diluted in absolute ethanol (20 mL) and distilled pyridine (20 mL). The reaction was heated up to reflux for 3 hours, then the volatiles were removed and the crude product extracted with EtOAc (10mL). The organic phase was washed with HCI (2x10mL, 1M), saturated solution of Na₂C0₃ (10mL) and brine (10mL). Then, the organic phase was dried over MgSO₄ and concentrated to dryness to give quantitatively the desired oxime ether **2b** as a white solid, which was used in the next step without purification. ¹H NMR (CDC1₃): 3.05 (2H, m), 3.69 (1H, m), 3.82 (3H, s), 3.83 (3H, s), 4.06 (3H, s), 6.10 (1H, d, J = 2.0 Hz), 6.27 (1H, d, J = 2.0 Hz), 6.76 (3H, m), 7.00 (2H, d, J = 8.5 Hz), 7.05 (1H, m), 7.29 (2H, d, J = 8.5 Hz). ¹³C NMR (CDCl₃): 29.7, 49.6, 55.8, 55.9, 62.8, 87.5, 89.8, 93.2, 101.5, 108.0, 113.9 (d, J = 20.9 Hz), 115.1 (d, J = 20.9 Hz), 121.9, 123.5, 128.5 (2C), 129.5 (d, J = 8.4 Hz), 131.1 (2C), 133.5, 140.2 (d, J = 7.0 Hz), 158.9, 159.7, 160.3, 162.7 (d, J = 245.4 Hz), 164.5.

**3a-(4-Chlorophenyl)-6,8-dimethoxy-3-(3-fluorophenyl)-8b-hydroxy-2,3,3a,8b-tetrahydro-1*H*-cyclopenta[*b*]benzofuran-1-one *O*-methyl oxime (2c).** Ketone **1c** (300mg, 0.66 mmol) and O-methylhydroxylamine hydrochloride (3.29 mmol) were diluted in absolute ethanol (12 mL) and distilled pyridine (12mL). The reaction was heated up to reflux for 2 hours, then the volatiles were removed and the crude product extracted with EtOAc (20mL). The organic phase was washed with HCl (2x10mL, 1M), saturated solution of Na₂C0₃ (10mL) and brine (10 mL). Then, the organic phase was dried over MgSO₄ and concentrated to dryness to give 296 mg (93%) of 2c as a white solid, which was used in the next step without purification. ¹H NMR (CDCl₃) : 2.73 (s, OH), 2.99-3.12 (2H, m), 3.66-3.72 (1H, m), 3.82 (3H, s), 3.83 (3H, s), 4.06 (3H, s), 6.10 (1H, d, J= 2Hz), 6.27 (1H, d, J = 2Hz), 6.73-6.79 (3H, m), 7.02-7.05 (1H, m), 7.06 (2H, d, J = 8.5 Hz), 7.14 (2H, d, J = 8.5 Hz). ¹³C NMR (CDCl₃) : 29.7, 49.7, 55.8, 55.9, 62.8, 87.5, 89.8, 93.2, 101.5, 108.0, 113.8 and 114.0 (CH), 115.0 and 115.2 (CH), 123.5, 128.1, 128.2, 129.4 and 129.5 (CH), 133.0, 133.6, 140.2 and 140.3 (C), 158.9, 159.7, 160.3, 161.4 and 163.9 (C), 164.4. LC-MS : calculated : 483.1 ; found : 466.0 (MH⁺-H₂O)⁺.

**1-Amino-3a-(4-bromophenyl)-6,8-dimethoxy-3-phenyl-2,3,3a,8b-tetrahydro-1*H-*cyclopenta[*b*]benzofuran-8b-ol (3a1 and 3a2).** Borane-tetrahydrofuran complex (20 mL, 20 mmol, 1M) was added dropwise to a solution of oxime **2a** (538 mg, 1.04 mmol) in THF (5 mL) at O°c. The mixture was heated under reflux for 12 hours, and cooled in an ice/water bath. A solution of NaOH (30 mL, 3 N) was carefully added, and the aqueous phase was extracted with CH₂Cl₂ (2 x 20 mL). The organic layer was washed with water (20 mL) and brine (20 mL), dried over MgSO₄ and concentrated to dryness. Purification by flash chromatography (Et₂O/MeOH : 70/30) provided 221 mg (44%) of the desired amines **3a1** and **3a2** as an inseparable mixture in a 30:70 ratio. ¹H NMR (CDCl₃): 2.02-2.12 (1H, m), 2.43-2.49 (1H, m), 3.57 (1H, dd, J = 5.9, 14.4 Hz), 3.79 (6H, s), 3.82 (1H, m), 6.06 (1H, d, J = 2 Hz), 6.21 (1H, d, J = 2.0 Hz), 7.02 (2H, d, J = 8.7 Hz), 6.99-7.11 (5H, m), 7.19 (2H, d, J = 8.7 Hz). ¹³C NMR (CDCl₃): 36.9, 52.0, 55.7, 55.8, 57.0, 86.4, 88.9, 92.5, 101.9, 112.1, 121.0, 126.6, 128.0, 128.1, 129.5, 130.2, 135.9, 138.3, 157.6, 159.7, 163.4.

**1-Amino-3a-(4-bromophenyl)-6,8-dimethoxy-3-(3-fluorophenyl)-8b-hydroxy-2,3,3a,8b-tetrahydro-1*H*-cyclopenta[*b*]benzofuran-8b-ol (3bl and 3b2)**. Borane-tetrahydrofuran complex (15 mL, 15 mmol, 1M) was added dropwise to a solution of methylether oxime 2b (1.60 g, 3.03 mmol) in dry THF (15 mL) at 0°C. After addition, the reaction was heated to reflux for 12 hours. At room temperature, a solution of NaOH (30 mL, 3 N) was added to stop the reaction and the aqueous phase was extracted with CH₂Cl₂ (2 x 20 mL). The cumulated organic phase was washed with demineralised water (20mL), brine (20 mL), dried over MgSO₄ and concentrated to dryness. The crude product was purified by chromatography (Et₂O/MeOH 70/30) to give 645 mg (41%) of the desired amines **3b1** and **3b2** as a mixture of diastereomers (ratio 5/95). ¹H NMR (CDCl₃): 2.05 (1H, m), 2.45 (1H, m), 3.57 (1H, m), 3.77 (7H, m), 6.03 (1H, d, J = 1.9 Hz), 6.17 (1H, d, J = 1.9 Hz), 6.74 (3H, m), 7.01 (3H, m), 7.18 (2H, d, J = 8.7 Hz). ¹³C NMR (CDC1₃): 36.5, 51.8, 55.7, 55.8, 57.1, 86.4, 88.9, 92.5, 101.8, 111.6, 113.4 (d, J = 21.3 Hz), 115.0 (d, J = 21.3 Hz), 121.2, 123.6, 129.3 (2C), 129.4, 130.3 (2C), 135.4, 140.9 (d, J = 7.3 Hz), 157.6, 159.6, 162.6 (d, J = 245.4 Hz), 163.5. IR (thin film): 3306, 2943, 2939, 1602, 1146, 691, 501 cm⁻¹.

**1-Amino-3a-(4-chlorophenyl)-6,8-dimethoxy-(3-fluorophenyl)-8b-hydroxy-3-2,3,3a,8b-tetrahydro-1*H*-cyclopenta[*b*]benzofuran-8b-ol (3c2).** Borane-tetrahydrofuran complex (8 mL, 8 mmol, 1M) was added dropwise to a solution of methylether oxime 2c (273 mg, 0.564 mmol) in dry THF (20mL) at 0°C. After addition, the reaction was heated to 65°C overnight. Again BH₃.THF (2mL) was added at room temperature, then heated up to 65°C for 8 hours. A solution of 5 M NaOH (5 mL) was added to stop the reaction and the aqueous phase was extracted with EtOAc (2 x 25mL). The cumulated organic phase was washed with brine (20 mL), dried over MgSO₄ and concentrated to dryness. The crude product was purified by chromatography (MeOH) to give 218 mg (84%) of the desired amine **3c2.** Rf (MeOH) = 0.35. ¹H NMR (CDCl₃) : 1.74 (s, NH₂), 2.00-2.09 (1H, m), 2.43-2.47 (1H, m), 3.55 (1H, dd, *J* = 5.8, 14.4 Hz), 3.79 (3H, s), 3.80 (3H, s), 3.81-3.86 (1H, m), 4.77 (s, OH), 6.06 (1H, d, *J* = 1.8Hz), 6.20 (1H, d, *J* = 1.8Hz), 6.72-6.79 (3H, m), 7.02-7.10 (5H, m). ¹³C NMR (CDCl₃) : 36.8, 51.8, 55.7, 55.8, 57.0, 86.5, 89.0, 92.6, 101.7, 111.9, 113.5 (d, J = 20.9 Hz), 115.1 (d, J = 21.6 Hz), 123.6, 127.4, 129.0, 129.4 (d, J = 8.1 Hz), 132.9, 135.0, 141.0 (d, J = 7.3 Hz), 157.7, 159.6, 162.6 (d, J = 244.7 Hz), 163.5.

**N-(3a-(4-Bromophenyl)-8b-hydroxy-6,8-dimethoxy-3-phenyl-2,3,3a,8b-tetrahydro-1*H*-cyclopenta[*b*]benzofuran-1-yl)formamide (FL22 and FL23).** A 3:7 mixture of amines **3a1** and **3a2** (130 mg, 0.27 mmol) was heated under reflux for 12 hours in THF (2 mL), ethyl formate (0.35 mL, 4.31 mmol) and a drop of acetic acid. Concentration and purification by flash chromatography (Et₂O /AcOEt : 6/4) afforded formamides **FL22** (11 mg, 8%) and **FL23** (60 mg, 44%) as white solids.

**FL22.** ¹H NMR (CDCl₃): 2.09 (1H, m), 2.82 (1H, m), 3.84 (7H, m), 4.98 (1H, m), 6.08 (1H, d, *J* = 2.0 Hz), 6.21 (1H, d, *J* = 2.0 Hz), 6.90-7.10 (7H, m), 7.15 (2H, d, *J* = 8.7 Hz), 8.07 (1H, s). ¹³C NMR (CDCl₃): 34.1, 53.7, 55.4, 55.5, 58.3, 88.9, 92.4, 92.6, 102.4, 106.4, 121.1, 126.4, 127.9 (2C), 128.3 (2C), 129.4 (2C), 129.8 (2C), 135.0, 138.6, 157.6, 161.0, 161.9, 163.9. IR (thin film): 3338, 2928, 1673, 1596, 700 cm⁻¹. LC-MS : calculated : 509.1 ; found : 492.0 (MH⁺-H₂O)⁺.

**FL23.** ¹H NMR (CDCl₃): 2.25 (1H, m), 2.70 (1H, m), 3.56 (1H, m), 3.76 (3H, s), 3.80 (3H, s), 4.69 (1H, m), 6.06 (1H, d, *J* = 2.0 Hz), 6.21 (1H, d, *J* = 2.0 Hz), 6.96 (2H, m), 7.02-7.08 (5H, m), 7.19 (2H, d, *J* = 8.8 Hz), 8.24 (1H, s). ¹³C NMR (CDCl₃): 35.4, 53.7, 53.9, 55.1, 55.3, 86.2, 88.6, 92.0, 101.7, 110.6, 120.7, 126.3, 127.5 (2C), 127.7 (2C), 129.3 (2C), 129.7 (2C), 135.1, 137.5, 157.6, 159.2, 161.6, 163.5. IR (thin film): 3585, 3386, 2944, 2839, 1666, 1694, 698 cm⁻¹. LC-MS : calculated: 509.1 ; found: 492.0 (MH⁺-H₂O)⁺.

***N*-(3*a*-(4-Bromophenyl)-8*b*-hydroxy-6,8-dimethoxy-3-phenyl-2,3,3*a*,8*b-*tetrahydro-1H-cyclopenta[*b*]benzofuran-1-yl)acetamide (FL27 and FL28).** Anhydride acetic (35 µL, 0.37 mmol) and DMAP (3 mg, 0.025 mmol) were added to a 3:7 mixture of amines **3a1** and **3a2** (60 mg, 0.12 mmol) in pyridine (1 mL). Concentration in vacuo and purification by flash chromatography (Et₂O /AcOEt 6/4) afforded formamides **FL27** (7 mg, 11%) and **FL28** (30 mg, 46%) as white solids.

**FL27.** ¹H NMR (CDCl₃): 1.97 (3H, s), 2.11 (1H, m), 2.73 (1H, m), 3.82 (3H, s), 3.85 (4H, m), 5.00 (1H, m), 6.09 (1H, d, *J* = 2.0 Hz), 6.20 (1H, d, *J* = 2.0 Hz), 7.00 (2H, d, *J* = 8.7 Hz), 7.08 (5H, m), 7.18 (2H, d, *J* = 8.7 Hz). ¹³C NMR (CDCl₃): 23.6, 34.2, 53.8, 55.9 (2C), 60.8, 89.2, 92.7, 92.8, 103.0, 106.6, 121.8, 126.7, 128.3 (2C), 128.9 (2C), 129.8 (2C), 130.2 (2C), 135.0, 139.1, 157.6, 161.4, 164.1, 190.8. IR (thin film): 3361, 2942, 1622, 700, 504 cm⁻¹. LC-MS : calculated : 523.1 ; found : 522.0 (M-H)⁻.

**FL28.** ¹H NMR (CDCl₃): 2.04 (3H, s), 2.21 (1H, m), 2.71 (1H, m), 3.61 (1H, m), 3.71 (3H, s), 3.76 (3H, s), 4.62 (1H, m), 5.99 (1H, d, *J* = 2.0 Hz), 6.15 (1H, d, *J* = 2.0 Hz), 6.90-7.10 (7H, m), 7.16 (2H, d, *J* = 8.5 Hz). ¹³C NMR (CDCl₃): 23.5, 35.9, 52.1, 55.7, 55.8 (2C), 87.4, 88.9, 92.5, 102.1, 110.3, 121.3, 126.5, 127.9 (2C), 128.0 (2C), 129.5 (2C), 130.3 (2C), 135.3, 137.9, 157.8, 159.7, 163.8, 171.6. IR (thin film): 3395, 3158, 2947, 1644, 699 cm⁻¹. LC-MS: calculated : 523.1 ; found : 506.0 (MH⁺-H₂O)⁺.

***N*-(3a-(4-Bromophenyl)-3-(3-fluorophenyl)-8b-hydroxy-6,8-dimethoxy-2,3,3a,8b-tetrahydro-1*H*-cyclopenta[*b*]benzofuran-1-yl)formamide (FL32)**. To a solution of diastereomeric amines **3b1** and **3b2** (120 mg, 0.24 mmol) in dry THF (2 mL) was added ethyl formate (0.31 mL, 3.84 mmol) and a drop of acetic acid. The reaction was heated to reflux for 12 hours. At room temperature, the volatiles were removed and the crude product purified by chromatography (AcOEt) to give 103 mg (79%) of **FL32** as a white solid. ¹H NMR (CDCl₃): 2.25 (1H, m), 2.81 (1H, m), 3.63 (1H, m), 3.76 (3H, s), 3.83 (3H, s), 4.73 (1H, m), 6.06 (1H, d, *J* = 2.0 Hz), 6.22 (1H, d, *J* = 2.0 Hz), 6.81 (3H, m), 7.07 (3H, m), 7.26 (2H, d, *J* = 8.8 Hz), 8.26 (1H, s). ¹³C NMR (CDCl₃): 36.0, 51.8, 54.3, 55.8, 55.9, 87.4, 88.9, 92.8, 102.0, 110.0, 113.6 (d, *J* = 20.9 Hz), 115.1 (d, *J* = 21.6 Hz), 121.8, 123.6, 129.2, 129.4 (2C), 130.6 (2C), 134.6, 140.4 (d, *J* = 7.3 Hz), 157.8, 159.7, 160.9, 162.6 (d, *J* = 245.4 Hz), 164.1. IR (thin film): 3306, 2943, 1599, 1147, 725 cm⁻¹. LC-MS : calculated : 527.1, found : 528.0 (M+H)⁺.

***N*-(3a-(4-Bromophenyl)-3-(3-fluorophenyl)-8b-hydroxy-6,8-dimethoxy-2,3,3a,8b-tetrahydro-1*H*-cyclopenta[*b*]benzofuran-1-yl)acetamide (FL33).** To a solution of diastereomeric amines **3b1** and **3b2** (115 mg, 0.23 mmol) in dry pyridine (2 mL) was added anhydride acetic (65 µL, 0.69 mmol) and DMAP (6 mg, 0.046 mmol). The reaction was stirred for 12 hours. Then, the volatiles were removed and the crude product purified by chromatography (Et₂O /AcOEt : 6/4) to give 79 mg (63%) of **FL33** as a white solid. ¹H NMR (CDCl₃): 2.04 (3H, s), 2.20 (1H, m), 2.74 (1H, m), 3.63 (1H, m), 3.72 (3H, s), 3.78 (3H, s), 4.62 (1H, m), 6.01 (1H, d, *J* = 2.0 Hz), 6.17 (1H, d, *J* = 2.0 Hz), 6.75 (3H, m), 7.02 (3H, m), 7.26 (2H, d, *J* = 8.8 Hz). ¹³C NMR (CDCl₃): 23.6, 36.0, 51.9, 55.8, 55.9 (2C), 87.8, 88.9, 92.7, 102.0, 110.0, 113.6 (d, *J* = 20.9 Hz), 115.1 (d, *J* = 21.6 Hz), 121.7, 123.7, 129.4 (2C), 129.5, 130.6 (2C), 134.8, 140.7 (d, *J* = 7.3 Hz), 157.8, 159.8, 162.7 (d, *J* = 245.4 Hz), 164.1, 170.1. IR (thin film): 3393, 3162, 2946, 2840, 1149, 704 cm⁻¹. LC-MS : calculated : 541.1, found : 542.0 (M+H)⁺.

***N*-(3a-(4-Bromophenyl)-3-(3-fluorophenyl)-8b-hydroxy-6,8-dimethoxy-2,3,3a,8b-tetrahydro-1*H*-cyclopenta[*b*]benzofuran-1-yl)propionamide (FL34).** To a solution of diastereomeric amines **3b1** and **3b2** (100 mg, 0.2 mmol) in dry pyridine (1mL) was added anhydride propionic (77 µL, 0.6 mmol) and DMAP (5 mg, 0.04 mmol). The reaction was stirred for 12 hours. Then, the volatiles were removed and the crude product purified by chromatography (Et₂O /AcOEt : 6/4) to give 95 mg (86%) of **FL34** as a white solid. ¹H NMR (CDCl₃): 1.18 (3H, t, *J* = 7.8 Hz), 2.16 (1H, m), 2.27 (2H, q, *J* = 7.8 Hz), 2.74 (1H, m), 3.62 (1H, m), 3.69 (3H, s), 3.77 (3H, s), 4.58 (1H, m), 5.99 (1H, d, *J* = 2.0 Hz), 6.16 (1H, d, *J* = 2.0 Hz), 6.74 (3H, m), 7.01 (3H, m), 7.20 (2H, d, *J* = 8.8 Hz). ¹³C NMR (CDCl₃): 10.2, 30.0, 36.0, 51.9, 55.7, 55.8 (2C), 87.8, 88.9, 92.7, 102.0, 110.0, 113.5 (d, *J* = 21.3 Hz), 115.1 (d, *J* = 21.6 Hz), 121.7, 123.7, 129.4 (2C), 129.5, 130.6 (2C), 134.9, 140.7 (d, *J* = 7.3 Hz), 157.8, 159.7, 162.7 (d, *J* = 245.4 Hz), 164.1, 173.8. IR (thin film): 3307, 2967, 1608, 1143, 732 cm⁻¹. LC-MS : calculated : 555.1, found : 556.0 (M+H)⁺.

***N*-(3a-(4-Bromophenyl)-3-(3-fluorophenyl)-8b-hydroxy-6,8-dimethoxy-2,3,3a,8b-tetrahydro-1*H*-cyclopenta[*b*]benzofuran-1-yl)-2-hydroxyacetamide (FL35).** EDCI (69 mg, 0.36 mmol), HOBt (49 mg, 0.36 mmol) and DIPEA (74 µL, 0.45 mmol) were added to a solution of glycolic acid (23 mg, 0.30 mmol) in dry DMF (1 mL) at 0°C. After 15 minutes, a solution of diastereomeric amines **3b1** and **3b2** (150 mg, 0.30 mmol) in dry DMF (1 mL) was added and the reaction was stirred for 24 hours at room temperature. The reaction was quenched by addition of EtOAc (10 mL) and washed with HCl (2x5 ml, 1M), saturated solution of NaHCO₃ (10 mL) and brine (10 mL). The organic phase was dried over MgSO₄ and concentrated to dryness. The crude product was purified by chromatography (EtOAc) to give 90 mg (51%) of **FL35** as a white solid. ¹H NMR (CDCl₃): 2.25 (1H, m), 2.72 (1H, m), 3.64 (1H, m), 3.67 (3H, s), 3.80 (3H, s), 4.69 (1H, m), 6.00 (1H, d, *J* = 1.9 Hz), 6.19 (1H, d, *J* = 1.9 Hz), 6.77 (3H, m), 6.99 (2H, d, *J* = 8.7 Hz), 7.05 (1H, m), 7.19 (2H, d, *J* = 8.7 Hz). ¹³C NMR (CDCl₃): 35.8, 52.0, 55.6, 55.8, 55.9, 62.5, 87.9, 89.0, 92.8, 102.0, 109.8, 113.6 (d, *J* = 20.9 Hz), 115.1 (d, *J* = 21.6 Hz), 121.8, 124.7, 129.3 (2C), 129.5 (d, *J* = 8.8 Hz), 130.7 (2C), 134.7, 140.4 (d, *J* = 7.3 Hz), 157.7, 159.8, 162.7 (d, *J* = 245.4 Hz), 164.2, 171.5. IR (thin film): 3382, 2928, 2841, 1147, 735 cm⁻¹. LC-MS : calculated : 557.1, found : 558.0 (M+H)⁺.

***N*-(3a-(4-Bromophenyl)-3-(3-fluorophenyl)-8b-hydroxy-6,8-dimethoxy-2,3,3a,8b-tetrahydro-1*H*-cyclopenta[*b*]benzofuran-1-yl)-2-(dimethylamino)acetamide (FL36)**. To a solution of diastereomeric amines **3b1** and **3b2** (150 mg, 0.30 mmol) in dry DMF (1 mL) was added EDCI (69 mg, 0.36 mmol), HOBt (49 mg, 0.36 mmol), DIPEA (124 µL, 0.75 mmol) and N,N'-dimethylglycine hydrochloride (42 mg, 0.30 mmol) at 0°C. The reaction was stirred for 24 hours at room temperature and was quenched by addition of EtOAc (10 mL). The organic phase was washed with HCl (2 x 5 mL, 1M), saturated solution of NaHCO₃ (10 mL) and brine (10 mL), dried over MgSO₄ and concentrated to dryness. The crude product was purified by chromatography (EtOAc) to give 90 mg (51%) of **FL36** as a white solid. ¹H NMR (CDCl₃): 2.25 (7H, m), 2.77 (1H, m), 2.86 (1H, d, *J =* 15.7 Hz), 2.97 (1H, d, *J* = 15.7 Hz), 3.66 (1H, m), 3.76 (3H, s), 3.84 (3H, s), 4.75 (1H, m), 6.07 (1H, d, *J* = 1.9 Hz), 6.24 (1H, d, *J* = 1.9 Hz), 6.83 (3H, m), 7.10 (1H, m), 7.14 (2H, d, *J* = 8.8 Hz), 7.29 (2H, d, *J* = 8.8 Hz). ¹³C NMR (CDCl₃): 35.8, 45.8 (2C), 51.9, 55.4, 55.6, 55.9, 63.8, 87.9, 88.9, 92.7, 102.2, 110.6, 113.5 (d, *J* = 20.9 Hz), 115.1 (d, *J* = 21.6 Hz), 121.6, 123.7, 129.4, 129.5 (2C), 130.6 (2C), 135.1, 140.8 (d, *J* = 7.3 Hz), 157.7, 159.7, 162.7 (d, *J* = 245.4 Hz), 164.0, 170.1. IR (thin film): 3359, 2946, 2839, 1598, 1148, 731 cm⁻¹. LC-MS : calculated : 584.1, found : 585.0 (M+H)⁺.

***N*-(3a-(4-Bromophenyl)-3-(3-fluorophenyl)-8b-hydroxy-6,8-dimethoxy-2,3,3a,8b-tetrahydro-1*H*-cyclopenta[*b*]benzofuran-1-yl)-2-(dimethylamino)-1,1-**dimethylurea (FL37). DIPEA (66 µL, 0.38 mmol), DMAP (3 mg) and dimethylcarbamoyl chloride (32 µL, 0.35 mmol) were successively added to a solution of diastereomeric amines **3b1** and **3b2** (160 mg, 0.32 mmol) in dry CH₂Cl₂ (2 mL) at 0°C. The reaction was stirred for 24 hours at room temperature, then CH₂Cl₂ (10 mL) was added. The organic phase was washed with HCl (10 mL, 1M), brine (10 mL), dried over MgSO₄ and concentrated to dryness. The crude product was purified by chromatography (EtOAc/MeOH 95/5) to afford 154 mg (84%) of FL37. ¹H NMR (CDCl₃): 2.19 (1H, m), 2.65 (1H, m), 2.87 (6H, s), 3.58 (1H, m), 3.64 (3H, s), 3.72 (3H, s), 4.40 (1H, m), 5.94 (1H, d, *J* = 2.0 Hz), 6.13 (1H, d, *J* = 2.0 Hz), 6.74 (3H, m), 7.02 (3H, m), 7.17 (1H, d, *J* = 8.8 Hz). ¹³C NMR (CDCl₃): 36.2 (2C), 36.7, 51.7, 55.5, 55.7, 57.3, 87.8, 88.8, 92.5, 102.1, 110.4, 113.3 (d, *J* = 20.9 Hz), 115.1 (d, *J* = 21.6 Hz), 121.3, 123.7, 129.2, 129.3 (2C), 130.3 (2C), 135.2, 141.1 (d, *J* = 7.3 Hz), 157.7, 158.4, 159.7, 162.7 (d, *J* = 245.4 Hz), 163.7. IR (thin film): 3233, 2937, 1627, 1147, 724 cm⁻¹. LC-MS : calculated: 570.1, found: 571.0 (M+H)⁺.

***N*-(3a-(4-Bromophenyl)-3-(3-fluorophenyl)-8b-hydroxy-6,8-dimethoxy-2,3,3a,8b-tetrahydro-1*H*-cyclopenta[*b*]benzofuran-1-yl)urea (FL40).** Trimethylsilyl isocyanate (9 µl, 0.11 mmol) was added to a solution of diastereomeric amines **3b1** and **3b2** (40 mg, 0.08 mmol) in dry CH₂Cl₂ (0.5 mL) and isopropanol (0.5 mL). The reaction was stirred for 4 hours at room temperature under argon atmosphere. After the volatiles were removed, the crude product was purified by chromatography (CH₂Cl₂/Et₂O : 98/2) to afford 25 mg (63%) of **FL40.** ¹H NMR (CDCl₃): 2.13 (1H, m), 2.56 (1H, m), 3.52 (1H, m), 3.68 (3H, s), 3.76 (3H, s), 4.51 (1H, m), 5.99 (1H, d, *J* = 2.0 Hz), 6.16 (1H, d, *J* = 2.0 Hz), 6.72 (3H, m), 7.00 (3H, m), 7.13 (2H, d, *J* = 8.5 Hz). ¹³C NMR (CDCl₃): 36.3, 51.2, 55.7, 55.8, 56.4, 87.7, 89.1, 92.7, 102.0, 110.5, 113.6 (d, *J* = 20.5 Hz), 115.1 (d, *J* = 21.6 Hz), 121.6, 123.7, 129.4 (3C), 130.5 (2C), 134.8, 140.7 (d, *J* = 7.7 Hz), 157.6 159.4, 159.7, 162.7 (d, *J* = 245.4 Hz), 163.9. IR (thin film): 3500, 3459, 3353, 3179, 2938, 1142, 809, 725 cm⁻¹. LC-MS : calculated : 542.1, found : 543.0 (M+H)⁺.

***N*-(3a-(4-Chlorophenyl)-3-(3-fluorophenyl)-8b-hydroxy-6,8-dimethoxy-2,3,3a,8b-tetrahydro-1*H*-cyclopenta[*b*]benzofuran-1-yl)formamide (FL41).** A solution of amine 3c2 (67 mg, 0.15 mmol) was heated under reflux for 20 hours in THF (5 mL), ethyl formate (0.20 mL, 2.35 mmol) and a drop of acetic acid. Concentration and purification by flash chromatography (Et₂O /AcOEt : 6/4) afforded 9 mg (12%) of formamide FL41 as a white solid. ¹H NMR (CDCl₃) : 2.29 (1H, m), 2.87 (1H, m), 3.68 (1H, dd, *J* = 5.8, 14.4 Hz), 3.80 (3H, s), 3.85 (3H, s), 4.79 (1H, m), 6.09 (1H, d, *J* = 1.9 Hz), 6.25 (1H, d, *J* = 1.9 Hz), 6.79-6.85 (3H, m), 7.06-7.14 (5H, m), 8.36 (1H, s). ¹³C NMR (CDCl₃) : 36.1, 52.0, 54.5, 55.8, 55.9, 87.6, 89.0, 92.8, 102.1, 109.9, 113.7 (d, *J =* 20.9 Hz), 115.2 (d, *J* = 22.0 HZ), 123.7, 127.8 (2C), 129.1 (2C), 129.5 (d, *J* = 9.2 Hz), 133.6, 134.0, 138.2 (d, *J* = 8.4 Hz), 157.9, 159.8, 160.9, 162.4 (d, *J* = 309.2 Hz), 164.3. LC-MS : calculated 483.1 ; found : 482.0 (M-H)⁻.

***N*-(3a-(4-Chlorophenyl)-3-(3-fluorophenyl)-8b-hydroxy-6,8-dimethoxy-2,3,3a,8b-tetrahydro-1*H*-cyclopenta[*b*]benzofuran-1-yl)-2-(dimethylamino)-1,1-dimethylurea (FL42).** DIPEA (23 µL, 0.13 mmol), DMAP (3 mg) and dimethylcarbamoyl chloride (11 µL, 0.12 mmol) were successively added to a solution of amine **3c2** (50 mg, 0.11mmol) in dry CH₂Cl₂ (1mL) at 0°C. The reaction was stirred overnight at room temperature, then the volatiles were removed and the crude product was purified by chromatography (EtOAc) to afford 45mg (77%) of the desired urea **FL42.** ¹H NMR (CDCl₃) : 2.20-2.29 (1H, m), 2.44 (s, OH), 2.68-2.74 (1H, m) 2.92 (6H, s), 3.64 (1H, dd, *J* = 5.9, 14.4 Hz), 3.68 (3H, s), 3.76 (3H, s), 4.44-4.50 (1H, m), 5.71 (d, *J* = 5.6 Hz, NH), 5.98 (1H, d, *J* = 1.9 Hz), 6.16 (1H, d, *J* = 1.9 Hz), 6.72-6.83 (3H, m), 7.00-7.02 (1H, m), 7.05 (2H, d, *J* = 8.8 Hz), 7.10 (2H, d, *J* = 8.8 Hz). ¹³C NMR (CDCl₃) : 36.4 (2C), 36.8, 51.9, 55.6, 55.8, 57.5, 88.1, 88.9, 92.6, 102.1, 110.3, 113.4 (s, *J* = 20.9 Hz), 115.2 (d, *J =* 21.6 Hz), 123.8, 127.5, 129.1, 129.4 (d, *J* = 8.1 Hz), 133.2, 134.6, 141.1 (d, *J* = 7.7 Hz), 157.8, 158.6, 159.8, 161.4, 163.8. LC-MS : calculated 526.2 ; found : 525.0 (M-H)⁻.

Methyl **(3a-(4-chlorophenyl)-3-(3-fluorophenyl)-8b-hydroxy-6,8-dimethoxy-2,3,3a,8b-tetrahydro-1*H*-cyclopenta[*b*]benzofuran-1-yl)carbamate (FL43).** DIPEA (23 µL, 0.13 mmol), DMAP (3 mg) and methylchloroformate (10 µL, 0.12 mmol) were successively added to a solution of amine **3c2** (50 mg, 0.11 mmol) in dry CH₂Cl₂ (1mL) at 0°C. The reaction was stirred overnight at room temperature, then the volatiles were removed and the crude product was purified by chromatography (EtOAc/pentane 5/5 to 8/2) to afford 41mg (72%) of the desired carbamate **FL43.** ¹H NMR (CDCl₃) : 2.14 (s, OH), 2.23-2.30 (1H, m), 2.63-2.69 (1H, m), 3.61 (1H, dd, *J* = 5.8, 14.2 Hz), 3.69 (3H, s), 3.72 (3H, s), 3.79 (3H, s), 4.47 (1H, m), 5.84 (brs, NH), 6.02 (1H, d, *J* = 1.9 Hz), 6.18 (1H, d, *J* = 1.9 Hz), 6.71-6.80 (3H, m), 7.01-7.08 (5H, m). ¹³C NMR (CDCl₃) : 36.2, 51.8, 52.4, 55.7, 55.9, 57.4, 87.4, 88.9, 92.6, 102.1, 110.2, 113.6 (d, *J* = 20.9 Hz), 115.2 (d, *J* = 21.6 Hz), 123.7, 127.6, 129.0, 129.4 (d, *J* = 8.1 Hz), 133.4, 134.2, 140.7 (d, *J* = 7.3 Hz), 157.0, 157.7, 159.7, 162.6 (d, *J* = 245.4 Hz), 164.0. LC-MS : calculated 513,1 ; found : 496.0 (MH⁺-H₂O)⁺.

***N*-(3a-(4-Chlorophenyl)-3-(3-fluorophenyl)-8b-hydroxy-6,8-dimethoxy-2,3,3a,8b-tetrahydro-1*H*-cyclopenta[*b*]benzofuran-1-yl)-2-(dimethylamino)acetamide (FL44).** To a solution of N,N'-dimethylglycine hydrochloride (18 mg, 0.13 mmol) in dry DMF (4 mL) were added EDCI (29 mg, 0.15 mmol) and HOBt (20 mg, 0.15 mmol) at 0°C. After 15 min, a solution of amine 3c2 (57 mg, 0.13 mmol) in dry DMF (2 mL) was added slowly at 0°C. The reaction was stirred for 24 hours at room temperature and was quenched by addition of EtOAc (10mL). The organic phase was washed with HCl (2 x 5 ml, 1M), saturated solution of NaHCO₃ (10 mL), brine (10 mL), dried over MgSO₄ and concentrated to dryness. The crude product was purified by chromatography (EtOAc) to give 34 mg (50%) of **FL44** as a white solid. ¹H NMR (CDCl₃) : 2.17 (6H, s), 2.72 (1H, m), 2.80 (1H, d, *J* = 15.6 Hz, C**H**H'), 2.90 (1H, d, *J* = 15.6 Hz, CH**H'**), 3.62 (1H, dd, *J* = 5.9, 14.4 Hz), 3.71 (3H, s), 3.79 (3H, s), 4.70 (1H, td, *J* = 7.0, 11.0 Hz), 6.03 (1H, d, *J* = 1.9 Hz), 6.19 (1H, d, *J* = 1.9 Hz), 6.81-6.71 (3H, m), 7.07-7.02 (1H, m), 7.10 (2H, d, *J* = 8.8 Hz), 7.16 (2H, d, *J* = 8.8 Hz), 7.99 (1H, d, *J* = 7.3 Hz, NH). ¹³C NMR (CDCl₃) : 35.8, 45.8 (2C), 51.9, 55.4, 55.6, 55.8, 63.8, 87.8, 88.9, 92.7, 102.2, 110.6, 113.6 (d, *J* = 20.9 Hz), 115.2 (d, *J* = 21.6 Hz), 123.7, 127.6, 129.2, 129.4 (d, *J* = 8.4 Hz), 133.4, 134.6, 140.8 (d, *J* = 7.3 Hz), 157.8, 159.7, 162.6 (d, *J* = 245.0 Hz), 163.9, 170.1. LC-MS : calculated 540.2 ; found: 523.0 (MH⁺-H₂O)⁺.

### Example 1: Neuroprotection against cisplatin-induced apoptosis

The *in vitro* neuroprotectant activity against neurotoxicity of an antineoplastic agent was assayed on apoptosis induced by cisplatin (2.5 µM) in primary cultures of mouse cerebellar neurons. Neuronal survival was measured by MTT assay.

### Cell culture

Cerebellar granular neurons were dissected from 7-day old mice as described previously (Benosman et al., 2007). Briefly, after enzymatic and mechanical dissociation, cells were plated at a density of 500 cells/mm² on 0.1 mg/ml polyornithine-pre-coated culture dishes and were grown at 37 °C in a humidified atmosphere (5% CO2 / 95% air). Plating culture medium contained DMEM (Dulbecco's modified Eagle's medium; Life Technology) supplemented with 10% heat-inactivated horse serum (Life Technology), 100 nM insulin (Life Technology), 50 mg/ml gentamycin (Life Technology) and 25 mM KCl. 48 h after plating, cells were switched to defined medium (called high-potassium or HK medium) containing DMEM supplemented with 10 nM insulin, 100 mg/ml human transferrin, 60 mM putrescine, 30 nM sodium selenite, and 50 mg/ml gentamycin. Neurons were considered as immature up to 2 days after plating and as mature 5 days after plating when they develop a dense neuritis network.

### Colorimetric MTT assay

Mature neurons were cultured in 96-well culture dishes (Costar) and incubated for 48 hours in presence of cisplatine (2.5 µM) and flavagline derivative (1 nM) (which were both added into the medium at the same time). A modified procedure of the original method (Mosmann, 1983) was used to measure mitochondrial activity (MTT assay) as described previously (Meng et al., 2009). Briefly, cultures were rinsed with DMEM and incubated for 1 h at 37 °C in freshly prepared culture medium containing 0.5 mg/ml of MTT (3-(4,5-dimethylthiazol-2-yl)-2,5-diphenyl tetrazolium bromide; Sigma). Medium was then removed and dark blue crystals formed during reaction were dissolved by adding 100 µl/well of 0.04 M HCl in isopropyl alcohol. Plates were stirred at room temperature to ensure that all crystals were dissolved and read on a Bio-Rad 680 micro-ELISA plate-reader, using a test wavelength of 490 nm and a non-specific wavelength of 650 nm for background absorbency. Results are given as percentage of survival, taking cultures grown in control (vehicle) medium as 100%.

### Results

Results of this experiment are shown in Figure 3. These results demonstrate that the flavagline derivatives of the invention, FL23 and FL37, are able to prevent or limit neuronal cell apoptosis, in particular cisplatin-induced apoptosis. In comparison with a flavagline derivative of the prior art (FL15), previously described as neuroprotective agent in the international patent application WO 03/045375, flavagline derivatives of the invention show an improved efficiency as neuroprotective agent against neurotoxicity of an antineoplastic agent.

### Example 2: Neuroprotection against MPP⁺ -induced apoptosis (in vitro model of Parkinson's disease)

The neuroprotectant activity was tested in a cellular model of Parkinson's disease on primary mesencephalic cultures of rat embryos treated by MPP⁺ (3µM) (Hoglinger et al. 2003).

### Experimental protocol

Postmitotic dopaminergic neurons from the ventral mesencephalon of embryonic day 15.5 Wistar rat embryos (CERJ, Le Genest St. Isle, France) were dissociated mechanically, plated onto polyethylenimine-precoated 24-well culture plates, grown in N5 medium supplemented with 5 mM glucose, 5% horse serum and 2.5% fetal calf serum, as described previously (Michel et al., 1989). After 3 days, fetal calf serum was reduced to 0.5% to limit astrocyte proliferation. All experiments were initiated at day 5 in N5 medium containing 1 % horse serum and 0.1 % fetal calf serum. For each experiment MPP⁺ (Sigma) was diluted to 10 mM in H₂O. Cells were fixed with 4% formaldehyde for immunocytochemistry. Dopaminergic neurons were visualized by immunocytochemical detection of tyrosine hydroxylase (TH). Cultures were incubated with the primary antibody (mouse anti-TH, Diasorin, Antony, France, 1/1000; mouse-anti-MAP-2, Chemicon, Hofheim, Germany, 1/200), then with a biotinylated anti-mouse IgG (Vectastain, Burlingame, CA, 1/100) and revealed with the avidin-biotin method (Vectastain).

### Results

Results of this experiment are shown in Figure 4. These results demonstrate that the flavagline derivatives of the invention, FL23, FL32 and FL37, are able to prevent or limit neuronal cell degeneration, in particular MPP⁺-induced neurodegeneration, a well-known model of Parkinson's disease.

### Example 3: Cytotoxicity on cancer cells

The *in vitro* cytotoxicity of flavagline derivatives was evaluated on a variety of human cancer cell lines from nasopharynx (KB), neutrophil (HL60 and HL60R), colon (HCT116), breast (MDA435 and MDA231), ovary (OV3), and prostate (PC3)-derived neoplasms by the MTS assay after a 72 h treatment. Results are shown in Figure 5.

The human cell line KB (oral epidermoid carcinoma) was grown in D-MEM medium supplemented with 10% fetal calf serum, in the presence of penicilline, streptomycine and fungizone in 75cm² flask under 5% CO2, whereas HCT116 (colon adenocarcinoma), HL60 (promyeocytic leukaemia) were grown in RPMI medium. Resistant HL60R cells were obtained by prolonged treatment with doxorubicin.

Cells were plated in 96-well tissue culture plates in 200µl medium and treated 24h later with compounds dissolved in DMSO with compound concentrations ranged 0.5nM to 10µM using a Biomek 2000 (Beckman). Controls received the same volume of DMSO (1% final volume). After 72h exposure MTS reagent (Promega) was added and incubated for 3h at 37°C: the absorbance was monitored at 490nm and results expressed as the inhibition of cell proliferation calculated as the ratio [(OD₄₉₀ treated/OD₄₉₀ control)×100]. For IC₅₀ determinations (50% inhibition of cell proliferation) experiments were performed in separate duplicate.

### Results

Results of this experiment are shown in Figure 5. These results demonstrate that the flavagline derivatives of the invention exhibit a significant cytotoxic activity on various cancer cell lines, even on the cell line resistant to doxorubicin.

### Example 4: Cardioprotection

The *in vitro* cardioprotectant activity against cardiotoxicity of an antineoplastic agent was assayed on H9C2 cardiomyocytes treated with doxorubicin. Apoptosis was measured by FACS assay. Results are shown in Figure 6

### Cell culture

The H9c2 cardioblast cell line derived from embryonic rat heart was obtained from American Type Culture Collection (Manassas, VA, USA). Cells were grown in Dulbecco's modified Eagle's medium (DMEM) supplemented with 10% fetal calf serum at 37 °C in 5% CO₂. The medium was changed every 2-3 days.

### Cell culture and experimental design of in vitro cardiotoxicity assay

H9c2 cells were plated for 24h in 100 mm Petri dishes at the density of 0,7×10⁴ cells/cm². Next, the cells were washed and cultured for 12h in glucose-free medium (Gibco, DMEM w- L-glutamine, w/o D-glucose, sodium pyruvate) supplemented with only 1% fetal calf serum to synchronize the cells. Cells were pretreated with FL compounds or their vehicle for 12h and then were treated with doxorubicin for an additional 14h. Cells were washed, and apoptosis was analyzed by fluorescence-activated cell sorting analysis (FACS-Calibur, Becton-Dickinson Biosciences). 0,5×10⁶ cells were harvested, washed with Annexin Binding Buffer (0,01 M HEPES, 0,14 M NaCl, 2,5 mM CaCl₂) and labeled with annexin V (dilution 1:50) and propidium iodide (6,7 µg/ml). All assays were performed at least in triplicate, and the results were analyzed by BD Cell Quest Pro software (Becton-Dickinson Biosciences, Le Pont De Claix, France).

### Results

Results of this experiment are shown in Figure 6. These results demonstrate that the flavagline derivatives of the invention are able to prevent or limit cardiomyocyte apoptosis, in particular doxorubicin-induced apoptosis.

### References

Benosman S, et al. Cell Death Differ. 2007; 14: 2047-57.
Berge, et al. J. Pharmaceutical Sciences, 1977, 66: 1-19.
Diedrichs N., et al. Eur J Org Chem. 2005, 9, 1731-1735.
Dobler M.R., et al. Tetrahedron Lett. 2001;42: 8281-8284.
Fahrig T., et al. Mol Pharm. 2005 ; 67 : 1544-1555.
Gerard B., et al. J Am Chem Soc 2004 ; 27 : 126: 13620-1.
Hoglinger GU, et al. J Neurochem. 2003; 86: 1297-1307
Kaley TJ, Deangelis LM. Br J Haematol. 2009; 145(1):3-14.
Kim S, et al. Anticancer Agents Med Chem 2006;6:319-45.
Meng X, et al. Cancer Res. 2009; 69: 5458-66.
Michel PP, et al. J Pharmacol Exp Ther. 1989;248:842-850.
Mosmann T. J Immunol Methods. 1983; 65: 55-63.
Proksch P, et al. Curr Org Chem 2001;5:923-38.
Walker M, Ni O. Am J Clin Oncol. 2007; 30: 82-92.

## Claims

1. A flavagline derivative of the formula (I) 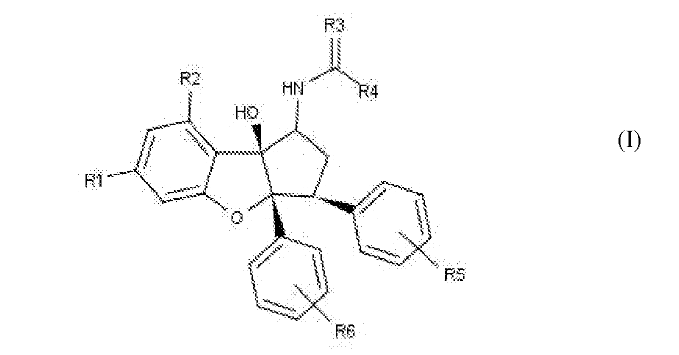 wherein
R¹ is alkoxy, optionally substituted, preferably selected from the group consisting of methoxy and a group -O-(CH₂)n-R⁷ wherein n is 1, 2, 3 or 4 and R⁷ is selected from the group consisting of hydroxyl, -NMe₂, -OCONMe₂, -OCONH₂ and morpholine;
R² is selected from the group consisting of hydrogen and (C₁-C₃)-alkoxy, preferably from hydrogen and methoxy;
R³ is selected from the group consisting of oxygen and sulphur;
R⁴ is selected from the group consisting of hydrogen, (C₁-C₃)-alkyl, optionally substituted, (C₁-C₃)-alkoxy, optionally substituted, (C₁-C₃)-hydroxyalkyl, a group - (CH₂)ₘ-N-R⁸R⁹, wherein m is 0, 1, 2 or 3, R⁸ and R⁹ are independently selected from the group consisting of hydrogen and (C₁-C₃)-alkyl;
R⁵ is selected from the group consisting of hydrogen and halogen, preferably from hydrogen and F;
R⁶ is halogen, preferably Br or Cl;
or an enantiomer thereof; or an enantiomeric mixture thereof; or any pharmaceutically acceptable salt thereof, for use for the prevention or treatment of a neuropathy selected from the group consisting of a neurodegenerative condition, a neuropathy induced by a neurotoxic agent and a neuropathy resulting from diabetes or HIV infection.

2. The flavagline derivative of claim 1, wherein the flavagline derivative presents the formula (II) 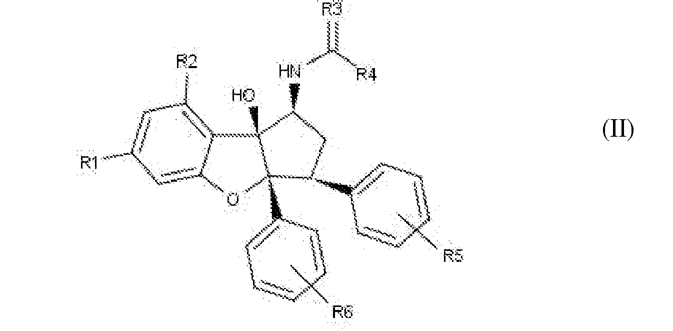 wherein the definitions of R¹, R², R³, R⁴, R⁵ and R⁶ are the same as in claim 1.

3. The flavagline derivative according to claim 1 or 2, wherein
R¹ and R² are methoxy;
R³ is oxygen;
R⁴ is selected from the group consisting of hydrogen, methyl, ethyl, methoxy, - CH₂OH, -NH₂, -NMe₂ and -CH₂NMe₂;
R⁵ is selected from the group consisting of hydrogen and halogen, preferably halogen; and
R⁶ is halogen, preferably Br or Cl.

4. The flavagline derivative according to any one of claims 1 to 3, wherein the neuropathy is a neuropathy induced by a neurotoxic agent and the neurotoxic agent is an antineoplastic agent.

5. A flavagline derivative of the formula (I) 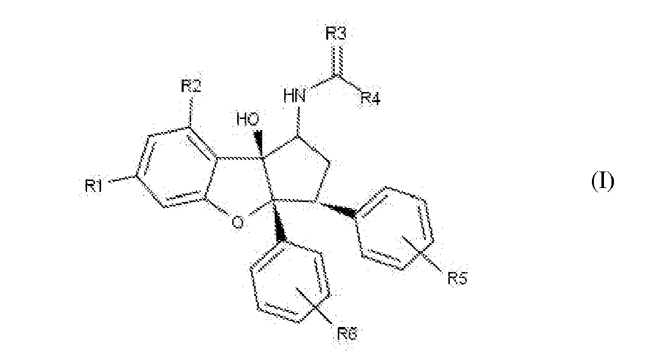 wherein
R¹ is alkoxy, optionally substituted, preferably selected from the group consisting of methoxy and a group -O-(CH₂)n-R⁷ wherein n is 1, 2, 3 or 4 and R⁷ is selected from the group consisting of hydroxyl, -NMe₂, -OCONMe₂, -OCONH₂ and morpholine;
R² is selected from the group consisting of hydrogen and (C₁-C₃)-alkoxy, preferably from hydrogen and methoxy;
R³ is selected from the group consisting of oxygen and sulphur;
R⁴ is selected from the group consisting of hydrogen, (C₁-C₃)-alkyl, optionally substituted, (C₁-C₃)-alkoxy, optionally substituted, (C₁-C₃)-hydroxyalkyl, a group - (CH₂)ₘ-N-R⁸R⁹, wherein m is 0, 1, 2 or 3, R⁸ and R⁹ are independently selected from the group consisting of hydrogen and (C₁-C₃)-alkyl;
R⁵ is selected from the group consisting of hydrogen and halogen, preferably from hydrogen and F;
R⁶ is halogen, preferably Br or Cl;
with the proviso that when R⁴ is hydrogen, then R⁵ is halogen,
or an enantiomer thereof; or an enantiomeric mixture thereof; or any pharmaceutically acceptable salt thereof.

6. The flavagline derivative according to claim 5, wherein the flavagline derivative presents the formula (II) 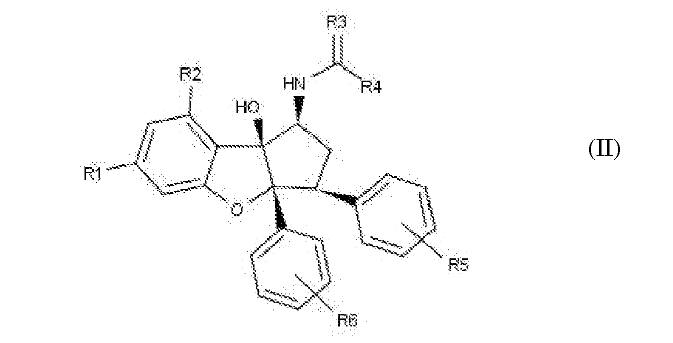 wherein the definitions of R¹, R², R³, R⁴, R⁵ and R⁶ are the same as in claim 5.

7. The flavagline derivative according to claim 5 or 6, wherein R¹ and R² are (C₁-C₃)-alkoxy, preferably methoxy, and R³ is oxygen.

8. The flavagline derivative according to any one of claims 5 to 7, wherein R⁴ is selected from the group consisting of hydrogen, methyl, ethyl, methoxy, -CH₂OH, -NH₂, - NMe₂ and -CH₂NMe₂.

9. The flavagline derivative according to any one of claims 5 to 8, wherein R⁵ is halogen, preferably F, and is in meta position, and R⁶ is selected from the group consisting of Br and Cl and is in para position.

10. The flavagline derivative according to any one of claims 5 to 9, wherein
R¹ and R² are methoxy;
R³ is oxygen;
R⁴ is selected from the group consisting of hydrogen, methyl, ethyl, methoxy, - CH₂OH, -NH₂, -NMe₂ and -CH₂NMe₂;
R⁵ is F and is in meta position; and
R⁶ is selected from the group consisting of Br and Cl and is in para position.

11. The flavagline derivative according to claim 10, wherein
R¹ and R² are methoxy;
R³ is oxygen;
R⁴ is selected from the group consisting of methyl, ethyl, methoxy, -CH₂OH, -NH₂, -NMe₂ and -CH₂NMe₂, preferably selected from the group consisting of methyl and ethyl;
R⁵ is hydrogen; and
R⁶ is selected from the group consisting of Br and Cl and is in para position.

12. The flavagline derivative according to any one of claims 5 to 11 as a medicament.

13. A pharmaceutical composition comprising a flavagline derivative according to any one of claims 5 to 11 and a pharmaceutically acceptable carrier and/or excipient.

14. A pharmaceutical composition comprising a flavagline derivative according to any one of claims 5 to 11 and an antineoplastic agent.

15. A product containing a flavagline derivative according to any one of claims 5 to 11 and an antineoplastic agent, as a combined preparation for simultaneous, separate or sequential use in the treatment of cancer.
